# EUROPEAN PATENT SPECIFICATION

(11) **EP 3 886 987 B1**
(45) Date of publication and mention of the grant of the patent: **15.11.2023**
(21) Application number: 19816924.5
(22) Date of filing: 28.11.2019
(51) Int. Cl.: A61P 31/18, A61P 35/00, C07D 513/04, A61K 31/554

(54) **COMPOUNDS USEFUL IN HIV THERAPY**
VERBINDUNGEN ZUR VERWENDUNG IN DER HIV-THERAPIE
COMPOSÉS UTILES DANS LA THÉRAPIE DU VIH

(30) Priority: 30.11.2018 US 201862773563 P
(43) Date of publication of application: 06.10.2021
(73) Proprietor: GlaxoSmithKline Intellectual Property Development Limited, Stevenage SG1 2NY (GB); VIIV Healthcare Company, Wilmington, DE 19808 (US); The University of North Carolina at Chapel Hill, Chapel Hill, North Carolina 27516 (US)
(72) Inventor: DE LA ROSA, Martha Alicia, Research Triangle Park, North Carolina 27709 (US); DUNHAM, Richard M, Research Triangle Park, North Carolina 27709 (US); MARGOLIS, David, Chapel Hill, North Carolina 27516 (US); TAI, Vincent Wing-Fai, Research Triangle Park, North Carolina 27709 (US); TANG, Jun, Research Triangle Park, North Carolina 27709 (US)
(74) Representative: Gladwin, Amanda Rachel
(86) International application number: PCT/IB2019/060267
(87) International publication number: WO 2020/110056

(56) References cited:
- EP-A1- 2 058 312
- WO-A2-2007/130626
- WO-A2-2011/050068
- WO-A2-2015/187998
- US-A1- 2015 307 499
- MITCHELL VAMOS ET AL: "Expedient Synthesis of Highly Potent Antagonists of Inhibitor of Apoptosis Proteins (IAPs) with Unique Selectivity for ML-IAP", ACS CHEMICAL BIOLOGY, vol. 8, no. 4, 19 April 2013 (2013-04-19), pages 725-732, XP055282328, ISSN: 1554-8929, DOI: 10.1021/cb3005512

## Description

### FIELD OF THE INVENTION

The present invention relates to a compound, pharmaceutical compositions, and use thereof in connection with individuals infected with HIV.

### SEQUENCE LISTING

This application contains sequences, listed in an electronic Sequence Listing entitled PR66692_Seq_List, 2 KB in size, created using Patent-In 3.5 on November 12, 2010.

### BACKGROUND OF THE INVENTION

Human immunodeficiency virus type 1 (HIV-1) infection leads to the contraction of acquired immune deficiency disease (AIDS). The number of cases of HIV continues to rise, and currently an estimated over thirty-five million individuals worldwide suffer from HIV infection e.g., http://www.sciencedirect.com/science/article /pii/S235230181630087X? via%3Dihub

Presently, long-term suppression of viral replication with antiretroviral drugs is the only option for treating HIV-1 infection. Indeed, the U.S. Food and Drug Administration has approved twenty-five drugs over six different inhibitor classes, which have been shown to greatly increase patient survival and quality of life. However, additional therapies are still believed to be required due to a number of issues including, but not limited to undesirable drug-drug interactions; drug-food interactions; non-adherence to therapy; drug resistance due to mutation of the enzyme target; and inflammation related to the immunologic damage caused by the HIV infection.

Currently, almost all HIV positive patients are treated with therapeutic regimens of antiretroviral drug combinations termed, highly active antiretroviral therapy ("HAART"). However, HAART therapies are often complex because a combination of different drugs must be administered often daily to the patient to avoid the rapid emergence of drug-resistant HIV-1 variants. Despite the positive impact of HAART on patient survival, drug resistance can still occur and the survival and quality of life are not normalized as compared to uninfected persons [Lohse Ann Intern Med 2007 146;87-95]. Indeed, the incidence of several non-AIDS morbidities and mortalities, such as cardiovascular disease, frailty, and neurocognitive impairment, are increased in HAART-suppressed, HIV-infected subjects [Deeks Annu Rev Med 2011;62:141-155]. This increased incidence of non-AIDS morbidity/mortality occurs in the context of, and is potentially caused by, elevated systemic inflammation related to the immunologic damage caused by HIV infection and residual HIV infection [Hunt J Infect Dis 2014][Byakagwa J Infect Dis 2014][Tenorio J Infect Dis 2014].

Modern antiretroviral therapy (ART) has the ability to effectively suppress HIV replication and improve health outcomes for HIV-infected persons, but is believed to not be capable of completely eliminating HIV viral reservoirs within the individual. HIV genomes can remain latent within mostly immune cells in the infected individual and may reactivate at any time, such that after interruption of ART, virus replication typically resumes within weeks. In a handful of individuals, the size of this viral reservoir has been significantly reduced and upon cessation of ART, the rebound of viral replication has been delayed [Henrich TJ J Infect Dis 2013][Henrich TJ Ann Intern Med 2014]. In one case, the viral reservoir was eliminated during treatment of leukemia and no viral rebound was observed during several years of follow-up [Hutter G N Engl J Med 2009]. These examples suggest the concept that reduction or elimination of the viral reservoir may be possible and can lead to viral remission or cure. As such, ways have been pursued to eliminate the viral reservoir, by direct molecular means, including excision of viral genomes with CRISPR/Cas9 systems, or to induce reactivation of the latent reservoir during ART so that the latent cells are eliminated. It is believed that reversal of latency is required to make latently infected cells vulnerable to clearance.

SMACm (Second Mitochondrial-derived Activator of Caspases) mimetics are a class of compounds that have recently entered clinical trials as potential cancer treatments. The drugs deplete and/or inhibit cellular inhibitor of apoptosis proteins (clAP) that act as anti-apoptotic proteins, thereby promoting the cell death of cancer cells. Antagonism and/or depletion of clAP also leads to activation of the non-canonical NF-kB signaling pathway, that may induce HIV expression and may enable elimination of HIV infected cells. In addition, SMAC mimetics may selectively promote the cell death of cells infected by HIV [Campbell Cell Host Microbe 2018] or HBV [Ebert Proc Nat Acad Sci 2013] by antagonizing anti-apoptotic proteins.

Recently, the targeting of the non-canonical NF-kB (ncNF-κB) pathway to reverse latency in cell line models was reported. The ncNF-κB pathway is typically activated by ligation of a subset of TNF receptor family members. In the steady state, a multimolecular complex with ubiquitin ligase activity consisting of TNF receptor-associated factor 2 (TRAF2), TRAF3, and cellular inhibitor of apoptosis protein-1 (clAP1) associates with the cytoplasmic portion of the unligated receptor and constitutively ubiquitinylates and degrades the NF-κB-inducing kinase (NIK). Upon receptor ligation, clAP1 ubiquitinylates TRAF3 and auto-ubiquitinylates, leading to proteasomal degradation of TRAF3 and clAP1, thereby disinhibiting NIK accumulation. NIK is constitutively active and, once accumulated, phosphorylates the inhibitor of κB kinase-α (IKKα) homodimer. The activated IKKα/IKKα homodimer then phosphorylates the inactive p100 form of NFκB2 leading to ubiquitinylation by Skp1-Cul1-F-box ubiquitin ligase (SCFβTrCP) and proteasomal cleavage of p100, releasing the active p52 subunit. p52 associates with RelB, and this heterodimer translocates into the nucleus to drive transcription from κB promoter elements. In addition to receptor ligation, ncNF-κB can be activated by signaling intermediates of the apoptosis cascade. Cleavage of the second mitochondrial activator of caspases (SMAC) from the mitochondrial membrane exposes the N-terminal motif Ala-Val-Pro-Ile, which binds specifically to the baculovirus intermediate repeat (BIR) domains of the IAP proteins. Such BIR binding in cIAP1/2 activates the ubiquitin ligase activity of the TRAF2:TRAF3:cIAP complex, inducing autoubiquitinylation and degradation of cIAP1/2, NIK accumulation, and activation of the ncNF-κB pathway. Binding of SMAC to the BIR domains of XIAP and ML-IAP antagonizes the caspase inhibition activities of these molecules, often overexpressed in tumor cells, leading to potentiation of apoptosis. As such the Ala-Val-Pro-Ile motif of SMAC has been the subject of significant attention in oncology, leading to discovery of a class of peptide mimetics that have SMAC-like activity, referred to as SMAC mimetics (SMACm). SMACm potently activate the ncNF-κB pathway and do not induce apoptosis in non-tumor cells, and as such are of interest to reverse HIV latency. See e.g., Richard Dunham et.al., The SMAC Mimetic AZD5582 is a Potent HIV Latency Reversing Agent, bioRxiv, May. 2, 2018; doi: http://dx.doi.org/10.1101/312447.

U.S. Patent No. 7,960,372 relates to bivalent diazo bicyclic SMAC mimetics that inhibit the activity of IAP.

### SUMMARY OF THE INVENTION

In one aspect, the invention provides a compound having the structure: 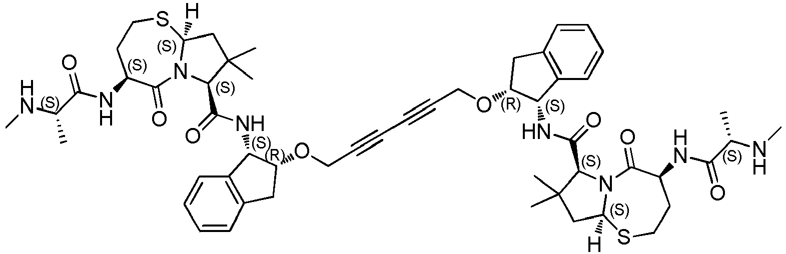 or a pharmaceutically acceptable salt thereof.

In another aspect, the invention provides a pharmaceutical composition comprising a compound as defined above or a pharmaceutically acceptable salt thereof and a pharmaceutically acceptable excipient.

In another aspect, the invention provides a compound as defined above or a pharmaceutically acceptable salt thereof for use in treating an HIV infection.

In another aspect, the invention provides a compound as defined above or a pharmaceutically acceptable salt thereof for use in depleting HIV infected cells.

In another aspect, the invention provides a compound as defined above or a pharmaceutically acceptable salt thereof and one or more additional agents active against HIV for use in depleting HIV infected cells. In certain aspects, these agents active against HIV are selected from the group consisting of anti-retroviral agents, latency reversing agents, and agents for clearance therapy.

These and other aspects are encompassed by the invention as set forth herein.

### BRIEF DESCRIPTION OF THE FIGURES

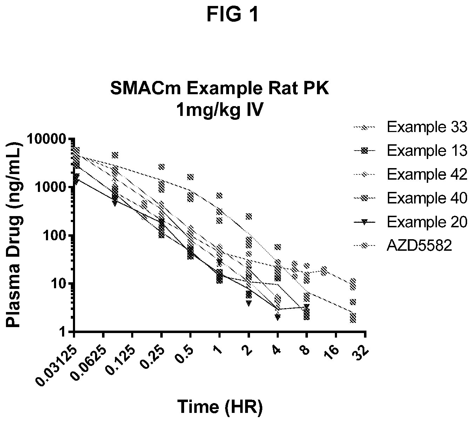

**FIG. 1** is a graph comparing rodent pharmacokinetic (PK) data of several compounds with that of SMACm AZD5582 PK data.

### DETAILED DESCRIPTION

The presently disclosed subject matter will now be described more fully hereinafter. However, many modifications and other embodiments of the presently disclosed subject matter set forth herein will come to mind to one skilled in the art to which the presently disclosed subject matter pertains having the benefit of the teachings presented in the foregoing descriptions. Therefore, it is to be understood that the presently disclosed subject matter is not to be limited to the specific embodiments disclosed and that modifications and other embodiments are intended to be included within the scope of the appended claims. In other words, the subject matter described herein covers all alternatives, modifications, and equivalents falling within the scope of the appended claims.

Unless otherwise defined, all technical and scientific terms used herein have the same meaning as commonly understood by one of ordinary skill in this field.

Apoptosis, a type of programmed cell death, plays an important role in maintaining homeostasis and regulating the number of cells in higher organisms. Abnormal apoptosis is involved in a number of diseases, including autoimmune disorders, degenerative diseases of the Central Nervous System, cancer, and viral infections, such as HIV. The family of Inhibitor of Apoptosis Proteins (lAPs) plays a key role in the suppression of proapoptotic signaling in mammalian cells. SMACm, which mimic a critical tetrapeptide sequence from the second mitochondria-derived activator of caspase, have been shown to disrupt the binding of lAPs with their functional partner and restore apoptotic response to proapoptotic stimuli in cells. Since the early 2000s, great effort has focused on the design and preparation of SMAC mimetics as IAP antagonists, particularly in promoting cell death in tumor cells and more recently in reversing HIV latency. Such investigations have explored the activation of the non-canonical NF-kB pathway (ncNF-kB) as a potential method by which SMAC mimics selectively deplete latent HIV cells. An example of an early SMAC mimetic is monomeric SBI-0637142, prepared by researchers at the Sanford-Burnham Medical Research Institute. In HIV depletion tests, SBI-0637142 was found to be potent in cell line assays, but did not exhibit activity in p100-p52 conversion or HIV caRNA induction in primary cells. Much work has also been directed to the development of bivalent mimetics, which are covalently linked momomeric SMAC mimetics. AstraZeneca's AZD5582 and Medivir's Birinapant TL32711 are examples of dimeric SMAC mimetics. In HIV latency reversal studies, Birinapant TL32711 was not potent in Jurkat, p100-p52 conversion, or HIV caRNA induction. Conversely, AZD5582 exhibited an increase in cell-associated HIV RNA expression in resting CD4+ T cells through Jurkat assay experiments, p100-p52 conversion studies, and HIV and CaRNA induction (Sampey et al. bioRxiv 312447). However, AZD5582 can also demonstrate tolerability issues.

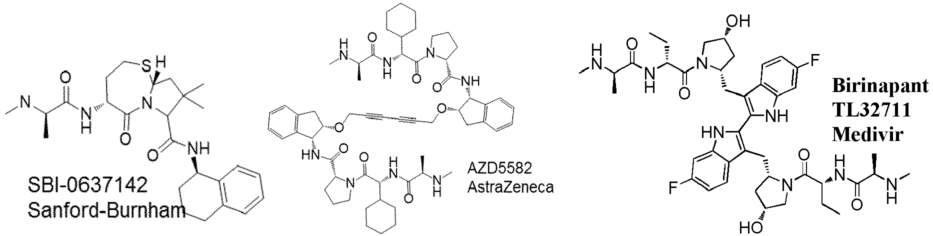

Disclosed herein are dimeric SMACm believed to be sufficiently potent and effective enough to activate ncNF-kB, reverse HIV latency in primary, unmodified human cells as single agents, and may have less off-target toxicity relative to other dimeric SMACm such as AZD5582, making them suitable for consideration for further development. In particular, the dimeric SMACm of the invention are capable of inducing HIV RNA expression in unstimulated, resting primary CD4+ T cells from HIV-infected donors whose viremia is completely suppressed by standard therapy. Other SMACm, specifically monomeric molecules or dimeric molecules with unoptimized linkers, are not believed to have this effect in these cells, the primary latent reservoir of persistent infection. Moreover, the dimeric SMACm of the invention are capable of a clinically measurable reversal of latency in two animal models (SIV-infected, antiretroviral suppressed rhesus macaques and HIV-infected ART-suppressed humanized mouse) as evidenced by intermittent plasma viremia that transiently emerges despite successful ongoing antiviral therapy

It is to be understood that the terminology used herein is for the purpose of describing particular embodiments only and is not intended to limit the scope of the present invention. In this specification and in the claims that follow, reference will be made to a number of terms that shall be defined to have the following meanings.

As used herein unless otherwise specified, "alkyl" refers to a monovalent saturated aliphatic hydrocarbyl group having from 1 to 14 carbon atoms and, in some embodiments, from 1 to 8 carbon atoms or 1 to 6 carbon atoms. "(Cx-Cy)alkyl" refers to alkyl groups having from x to y carbon atoms. The term "alkyl" includes, by way of example, linear and branched hydrocarbyl groups such as methyl (CH₃), ethyl (CH₃CH₂), *n*-propyl (CH₃CH₂CH₂), isopropyl ((CH₃)₂CH), *n*-butyl (CH₃CH₂CH₂CH₂), isobutyl ((CH₃)₂CHCH₂), *sec*-butyl ((CH₃)(CH₃CH₂)CH), *t*-butyl ((CH₃)₃C), *n*-pentyl (CH₃CH₂CH₂CH₂CH₂), and neopentyl ((CH₃)₃CCH₂). It should be noted that the recitation of e.g., (C₁-C₁₂) alkyl also encompasses ranges within this group e.g., (C₁-C₆)alkyl.

"AUC" refers to the area under the plot of plasma concentration of drug (not logarithm of the concentration) against time after drug administration.

"EC₅₀" refers to the concentration of a drug that gives half-maximal response.. Sometimes, it is also converted to the pEC₅₀ scale (-log IC₅₀), in which higher values indicate exponentially greater potency.

"IC₅₀" refers to the half-maximal inhibitory concentration of a drug. Sometimes, it is also converted to the pIC₅₀ scale (-log IC₅₀), in which higher values indicate exponentially greater potency.

"Compound" and"chemical entity" as used herein refers to a compound as defined above, and any forms of the compound including the racemates, stereoisomers, and tautomers of the compound or compounds.

"Polymorphism" refers to when two or more clearly different phenotypes exist in the same population of a species where the occurrence of more than one form or morph. In order to be classified as such, morphs must occupy the same habitat at the same time and belong to a panmictic population (one with random mating).

"Protein binding" refers to the binding of a drug to proteins in blood plasma, tissue membranes, red blood cells and other components of blood.

"Protein shift" refers to determining a binding shift by comparing the EC₅₀ values determined in the absence and presence of human serum.

"Racemates" refers to a mixture of enantiomers. In an embodiment of the invention, the compound as defined above, or pharmaceutically acceptable salts thereof, are enantiomerically enriched with one enantiomer wherein all of the chiral carbons referred to are in one configuration. In general, reference to an enantiomerically enriched compound or salt, is meant to indicate that the specified enantiomer will comprise more than 50% by weight of the total weight of all enantiomers of the compound or salt.

"Solvate" or "solvates" of a compound refer to those compounds, as defined above, which are bound to a stoichiometric or nonstoichiometric amount of a solvent. Solvates of a compound includes solvates of all forms of the compound. In certain embodiments, solvents are volatile, nontoxic, and/or acceptable for administration to humans in trace amounts. Suitable solvates include water.

"Stereoisomer" or "stereoisomers" refer to compounds that differ in the chirality of one or more stereocenters. Stereoisomers include enantiomers and diastereomers.

"Tautomer" refer to alternate forms of a compound that differ in the position of a proton, such as enol/keto and imine/enamine tautomers, or the tautomeric forms of heteroaryl groups containing a ring atom attached to both a ring NH moiety and a ring =N moiety such as pyrazoles, imidazoles, benzimidazoles, triazoles, and tetrazoles.

"Pharmaceutically acceptable salt" refers to pharmaceutically acceptable salts derived from a variety of organic and inorganic counter ions well known in the art and include, by way of example only, sodium, potassium, calcium, magnesium, ammonium, and tetraalkylammonium, and when the molecule contains a basic functionality, salts of organic or inorganic acids, such as hydrochloride, hydrobromide, tartrate, mesylate, acetate, maleate, and oxalate. Suitable salts include those described in P. Heinrich Stahl, Camille G. Wermuth (Eds.), Handbook of Pharmaceutical Salts Properties, Selection, and Use; 2002.

"Patient" or "subject" refers to mammals and includes humans and nonhuman mammals.

"Treating" or "treatment" of a disease in a patient refers to 1) preventing the disease from occurring in a patient that is predisposed or does not yet display symptoms of the disease; 2) inhibiting the disease or arresting its development; or 3) ameliorating or causing regression of the disease.

As indicated above, "treatment" of a disorder includes prevention of the disorder. A person of ordinary skill in the art will appreciate that "prevention" is not an absolute term. In medicine, "prevention" is understood to refer to the prophylactic administration of a drug to substantially diminish the likelihood or severity of a disorder or biological manifestation thereof, or to delay the onset of such disorder or biological manifestation thereof.

The terms "intermittent" or "intermittently" as used herein means stopping and starting at either regular or irregular intervals. As used herein, the term "viral infection" describes a diseased state in which a virus invades healthy cells, uses the cell's reproductive machinery to multiply or replicate and ultimately lyse the cell resulting in cell death, release of viral particles and the infection of other cells by the newly produced progeny viruses. Latent infection by certain viruses is also a possible result of viral infection.

As used herein, the term "treating viral infections" means to inhibit the replication of the particular virus, to inhibit viral transmission, and to ameliorate or alleviate the symptoms of the disease caused by the viral infection. The treatment is considered "therapeutic" if there is a reduction in viral load, decrease in mortality and/or morbidity. "Preventing viral infections" means to prevent the virus from establishing itself in the host. A treatment is considered "prophylactic" if the subject is exposed to the virus, but does not become infected with the virus as a result of treatment.

As used herein "latency" means a concept describing 1) the dormant state of viral activity within a population of cells, wherein viral production, viral packaging, and host cell lysis does not occur, or occurs at a very low frequency, or 2) the down-regulation or absence of gene expression within an infected cell.

As used herein, "reversing latent HIV infection" refers to a treatment that upregulates the expression of integrated HIV genomes within latently infected cells, such as the agent that activates the non-canonical NF-kB pathway, leading to susceptibility of the infected cell to virally-induced cell death or immunologic clearance. As used herein, "depleting latent HIV infection" refers to the clearance of latently HIV-infected cells that may follow the reversal of HIV latency by reagents such as those that activate the non-canonical NF-kB pathway.

In certain embodiments, the latent HIV infected cells are resting CD4⁺ T cells.

Where specific compounds or generic formulas are drawn that have aromatic rings, such as aryl or heteroaryl rings, then it will understood by one of still in the art that the particular aromatic location of any double bonds are a blend of equivalent positions even if they are drawn in different locations from compound to compound or from formula to formula. For example, in the two pyridine rings (A and B) below, the double bonds are drawn in different locations, however, they are known to be the same structure and compound:

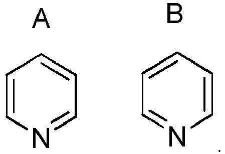

The present invention includes a compound as well as its pharmaceutically acceptable salts. Accordingly, the word "or" in the context of "a compound or a pharmaceutically acceptable salt thereof" is understood to refer to either: 1) a compound alone or a compound and a pharmaceutically acceptable salt thereof (alternative), or 2) a compound and a pharmaceutically acceptable salt thereof (in combination).

The compound encompassed by the present invention is shown in the following Table 1:

**Table 1**

| **Compound No** | **Compound structure** | **Compound name** |
|---|---|---|
| 13 | 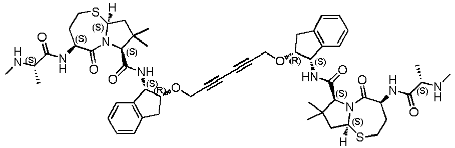 | (4S,4'S,7S,7'S,9aS,9a'S)-N,N'-((1S,1'S,2R,2'R)-(hexa-2,4-diyne-1,6-diylbis(oxy))bis(2,3-dihydro-1H-indene-2,1-diyl))bis(8,8-dimethyl-4-((S)-2-(methylamino)propanamido)-5-oxooctahydropyrrolo[2,1-b][1,3]thiazepine-7-carboxamide) |

Pharmaceutically acceptable salts are also within the scope of the invention with respect to the compound set forth herein. Most preferably, the compound may be present generically as hydrochloride (i.e., HCl salts), e.g., more specifically a dihydrochloride, (2 HCl) salt. Also within the scope of the invention is the compound present as a single species, including pharmaceutically acceptable salts thereof, as well the compound in free base form.

In accordance with one embodiment of the present invention, there is provided a pharmaceutical composition comprising a compound as defined above or a pharmaceutically acceptable salt thereof, and a pharmaceutically acceptable excipient. In a further embodiment, the compound is present in amorphous form. In a further embodiment, the pharmaceutical composition is in a tablet form. In a further embodiment, the compound is present as a spray dried dispersion. In a further embodiment, the composition is present in nanoparticulate form, e.g., particles between 1 and 100 nanometers in size.

In accordance with one embodiment of the present invention, there is provided a compound as defined above or pharmaceutically acceptable salt thereof for use in treating an HIV infection.

In accordance with one embodiment of the present invention, there is provided a pharmaceutical composition as described herein for use in treating an HIV infection.

Furthermore, the compound of the invention can exist in particular geometric or stereoisomeric forms. The invention contemplates all such compounds, including cis- and trans-isomers, (-)- and (+)-enantiomers, (R)- and (S)-enantiomers, diastereomers, (D)-isomers, (L)-isomers, the racemic mixtures thereof, and other mixtures thereof, such as enantiomerically or diastereomerically enriched mixtures, as falling within the scope of the invention. All such isomers, as well as mixtures thereof, are intended to be included in this invention.

Optically active (R)- and (S)-isomers and d and I isomers can be prepared using chiral synthons or chiral reagents, or resolved using conventional techniques. If, for instance, a particular enantiomer of a compound of the present invention is desired, it can be prepared by asymmetric synthesis, or by derivatization with a chiral auxiliary, where the resulting diastereomeric mixture is separated and the auxiliary group cleaved to provide the pure desired enantiomers. Alternatively, where the molecule contains a basic functional group, such as an amino group, or an acidic functional group, such as a carboxyl group, diastereomeric salts can be formed with an appropriate optically active acid or base, followed by resolution of the diastereomers thus formed by fractional crystallization or chromatographic means known in the art, and subsequent recovery of the pure enantiomers. In addition, separation of enantiomers and diastereomers is frequently accomplished using chromatography employing chiral, stationary phases, optionally in combination with chemical derivatization (e.g., formation of carbamates from amines).

In another embodiment of the present invention, there is provided a compound as defined above, or a pharmaceutically acceptable salt thereof for use in medical therapy.

In another embodiment of the present invention, there is provided a compound as defined above, or a pharmaceutically acceptable salt thereof for use in treating an HIV infection.

In one embodiment, the pharmaceutical formulation containing a compound as defined above, or a salt thereof is a formulation adapted for parenteral administration. In another embodiment, the formulation is a long-acting parenteral formulation. In a further embodiment, the formulation is a nano-particle formulation.

The compound of the present invention and its salts, solvates, or other pharmaceutically acceptable derivatives thereof, may be employed alone or in combination with other therapeutic agents. Therefore, in other embodiments, the treating and/or preventing an HIV infection in a subject may in addition to administration of a compound as defined above may further comprise administration of one or more additional pharmaceutical agents active against HIV.

In such embodiments, the one or more additional agents active against HIV is selected from the group consisting of anti-retroviral agents, latency reversing agents, and agents for clearance therapy.

In other embodiments, the one or more additional agents active against HIV is selected from the group consisting of nucleotide reverse transcriptase inhibitors, non-nucleotide reverse transcriptase inhibitors, protease inhibitors, entry inhibitors, attachment and fusion inhibitors, integrase inhibitors, maturation inhibitors, CXCR4 and/or CCR5 inhibitors, histone deacetylase inhibitors, histone crotonyl transferase inhibitors, protein kinase C agonists, proteasome inhibitors, TLR7 agonists, bromodomain inhibitors, and neutralizing antibodies, and combinations thereof.

In certain embodiments, the one or more additional agents active against HIV is selected from the group consisting of zidovudine, didanosine, lamivudine, zalcitabine, abacavir, stavudine, adefovir, adefovir dipivoxil, fozivudine, todoxil, emtricitabine, alovudine, amdoxovir, elvucitabine, nevirapine, delavirdine, efavirenz, loviride, immunocal, oltipraz, capravirine, lersivirine, GSK2248761, TMC-278, TMC-125, etravirine, saquinavir, ritonavir, indinavir, nelfinavir, amprenavir, fosamprenavir, brecanavir, darunavir, atazanavir, tipranavir, palinavir, lasinavir, enfuvirtide, T-20, T-1249, PRO-542, PRO-140, TNX-355, BMS-806, BMS-663068 and BMS-626529, 5-Helix, raltegravir, elvitegravir, dolutegravir,cabotegravir, bictegravir, vicriviroc (Sch-C), Sch-D, TAK779, maraviroc, TAK449, didanosine, tenofovir, lopinavir, darunavir, vorinostat, panobinostat, romidepin, valpronic acid, mocetinostat, sodium corotonate, bryostatin, ingenol B, disulforam, GS-9620, JQ1, iBET151, , bortezomib, epigallocatechin gallate, salinosporamide A, carfilzomib, broadly neutralizing antibodies (bNAb), eCD4-Ig, CD4-Ig, and dual-affinity re-targeting (DART) proteins.

As such, the compound of the present invention and any other pharmaceutically active agent(s) may be administered together or separately and, when administered separately, administration may occur simultaneously or sequentially, in any order. The amounts of the compound of the present invention and the other pharmaceutically active agent(s) and the relative timings of administration will be selected in order to achieve the desired combined therapeutic effect. The administration in combination of a compound of the present invention and salts, solvates, or other pharmaceutically acceptable derivatives thereof with other treatment agents may be in combination by administration concomitantly in: (1) a unitary pharmaceutical composition including both compounds; or (2) separate pharmaceutical compositions each including one of the compounds. Alternatively, the combination may be administered separately in a sequential manner wherein one treatment agent is administered first and the other second or vice versa. Such sequential administration may be close in time or remote in time. The amounts of the compound of the invention or salts thereof and the other pharmaceutically active agent(s) and the relative timings of administration will be selected in order to achieve the desired combined therapeutic effect.

In addition, the compound of the present invention may be used in combination with one or more other agents that may be useful in the treatment of HIV. They agents may include anti-retroviral agents, latency reversing agents, and agents for clearance therapy. Several examples of anti-retroviral agents are provided below:
Nucleotide reverse transcriptase inhibitors such as zidovudine, didanosine, lamivudine, zalcitabine, abacavir, stavudine, adefovir, adefovir dipivoxil, fozivudine, todoxil, emtricitabine, alovudine, amdoxovir, elvucitabine, and similar agents;
Non-nucleotide reverse transcriptase inhibitors (including an agent having anti-oxidation activity such as immunocal, oltipraz, etc.) such as nevirapine, delavirdine, efavirenz, loviride, immunocal, oltipraz, capravirine, lersivirine, GSK2248761, TMC-278, TMC-125, etravirine, and similar agents;
Protease inhibitors such as saquinavir, ritonavir, indinavir, nelfinavir, amprenavir, fosamprenavir, brecanavir, darunavir, atazanavir, tipranavir, palinavir, lasinavir, and similar agents;
Entry, attachment and fusion inhibitors such as enfuvirtide (T-20), T-1249, PRO-542, PRO-140, TNX-355, BMS-806, BMS-663068 and BMS-626529, 5-Helix and similar agents; Integrase inhibitors such as raltegravir, elvitegravir, dolutegravir, cabotegravir, bictegravir and similar agents;
Maturation inhibitors such as PA-344 and PA-457, and similar agents; and CXCR4 and/or CCR5 inhibitors such as vicriviroc (Sch-C), Sch-D, TAK779, maraviroc (UK 427,857), TAK449, as well as those disclosed in WO 02/74769, PCT/US03/39644, PCT/US03/39975, PCT/US03/39619, PCT/US03/39618, PCT/US03/39740, and PCT/US03/39732, and similar agents.

Further examples where the compound of the present invention may be used in combination with one or more agents useful in the prevention or treatment of HIV are found in Table 2.

**Table 2:**

| **FDA Approval** | **Brand Name** | **Generic Name** | **Manufacturer** |
|---|---|---|---|
| ***Nucleoside Reverse Transcriptase Inhibitors (NRTIs)*** | | | |
| 1987 | Retrovir | zidovudine, azidothymidine, AZT, ZDV | GlaxoSmithKline |
| 1991 | Videx | didanosine, dideoxyinosine, ddl | Bristol-Myers Squibb |
| 1992 | Hivid | zalcitabine, dideoxycytidine, ddC | Roche Pharmaceuticals |
| 1994 | Zerit | stavudine, d4T | Bristol-Myers Squibb |
| 1995 | Epivir | lamivudine, 3TC | GlaxoSmithKline |
| 1997 | Combivir | lamivudine + zidovudine | GlaxoSmithKline |
| 1998 | Ziagen | abacavir sulfate, ABC | GlaxoSmithKline |
| 2000 | Trizivir | abacavir+ lamivudine+ zidovudine | GlaxoSmithKline |
| 2000 | Videx EC | enteric coated didanosine, ddl EC | Bristol-Myers Squibb |
| 2001 | Viread | tenofovir disoproxil fumarate, TDF | Gilead Sciences |
| 2003 | Emtriva | emtricitabine, FTC | Gilead Sciences |
| 2004 | Epzicom | abacavir+ lamivudine | GlaxoSmithKline |
| 2004 | Truvada | emtricitabine + tenofovir disoproxil fumarate | Gilead Sciences |

| ***Non-Nucleosides Reverse Transcriptase Inhibitors (NNRTIs)*** | | | |
|---|---|---|---|
| 1996 | Viramune | nevirapine, NVP | Boehringer Ingelheim |
| 1997 | Rescriptor | delavirdine, DLV | Pfizer |
| 1998 | Sustiva | efavirenz, EFV | Bristol-Myers Squibb |
| 2008 | Intelence | Etravirine | Tibotec Therapeutics |

| ***Protease Inhibitors (PIs)*** | | | |
|---|---|---|---|
| 1995 | Invirase | saquinavir mesylate, SQV | Roche Pharmaceuticals |
| 1996 | Norvir | ritonavir, RTV | Abbott Laboratories |
| 1996 | Crixivan | indinavir, IDV | Merck |
| 1997 | Viracept | nelfinavir mesylate, NFV | Pfizer |
| 1997 | Fortovase | saquinavir (no longer marketed) | Roche Pharmaceuticals |
| 1999 | Agenerase | amprenavir, APV | GlaxoSmithKline |
| 2000 | Kaletra | lopinavir+ ritonavir, LPV/RTV | Abbott Laboratories |
| 2003 | Reyataz | atazanavir sulfate, ATV | Bristol-Myers Squibb |
| 2003 | Lexiva | fosamprenavir calcium, FOS-APV | GlaxoSmithKline |
| 2005 | Aptivus | tripranavir, TPV | Boehringer Ingelheim |
| 2006 | Prezista | Darunavir | Tibotec Therapeutics |

| ***Fusion Inhibitors*** | | | |
|---|---|---|---|
| 2003 | Fuzeon | Enfuvirtide, T-20 | Roche Pharmaceuticals & Trimeris |

| ***Entry Inhibitors*** | | | |
|---|---|---|---|
| 2007 | Selzentry | Maraviroc | Pfizer |

| ***Integrase Inhibitors*** | | | |
|---|---|---|---|
| 2007 | Isentress | Raltegravir | Merck |
| 2013 | Tivicay | Dolutegravir | ViiV Healthcare |
| --- | --- | Cabotegravir | |

The present invention may be used in combination with other agents that induce HIV expression, such as latency reversing agents. Several latency reversing agents include, but are not limited to, the following: histone deacetylase inhibitors (e.g., vorinostat, panobinostat, romidepin), histone crotonyl transferase inhibitors (sodium corotonate), protein kinase C agonists (e.g., bryostatin, ingenol B), disulfiram, TLR7 agonists (e.g., GS-9620), bromodomain inbhibitors (e.g., JQ1, iBET151). Many of these agents are described in further detail below.

The present invention may be used in combination with other agents that induce HIV expression, such as agents for clearance therapy. Several examples of agents for clearance therapy, or of immunological combinations for clearance, include, but are not limited to, the following: neutralizing and broadly neutralizing antibodies (bNAb), eCD4-Ig, CD4-Ig, and dual-affinity re-targeting (DART) proteins.

The scope of combinations of the compound of this invention with HIV agents is not limited to those mentioned above, but includes in principle any combination with any pharmaceutical composition useful for the treatment and/or prevention of HIV. As noted, in such combinations the compound of the present invention and other HIV agents may be administered separately or in conjunction. In addition, one agent may be prior to, concurrent to, or subsequent to the administration of other agent(s).

The present invention may be used in combination with one or more agents useful as pharmacological enhancers as well as with or without additional compounds for the prevention or treatment of HIV. Examples of such pharmacological enhancers (or pharmakinetic boosters) include, but are not limited to, ritonavir, GS-9350 (cobicistat), and SPI-452.

Ritonavir is 10-hydroxy-2-methyl-5-(1-methyethyl)-1-1[2-(1-methylethyl)-4-thiazolyl]-3,6-dioxo-8,11-bis(phenylmethyl)-2,4,7,12-tetraazatridecan-13-oic acid, 5-thiazolylmethyl ester, [5S-(5S*,8R*,10R*,11R*)] and is available from Abbott Laboratories of Abbott park, Illinois, as Norvir. Ritonavir is an HIV protease inhibitor indicated with other antiretroviral agents for the treatment of HIV infection. Ritonavir also inhibits P450 mediated drug metabolism as well as the P-gycoprotein (Pgp) cell transport system, thereby resulting in increased concentrations of active compound within the organism.

GS-9350 (cobicistat) is a compound being developed by Gilead Sciences of Foster City California as a pharmacological enhancer.

SPI-452 is a compound being developed by Sequoia Pharmaceuticals of Gaithersburg, Maryland, as a pharmacological enhancer.

In one embodiment of the present invention, a compound as defined above is used in combination with ritonavir. In one embodiment, the combination is an oral fixed dose combination. In another embodiment, the compound as defined above is formulated as a long acting parenteral injection and ritonavir is formulated as an oral composition. In one embodiment, a kit containing the compound as defined above is formulated as a long acting parenteral injection and ritonavir formulated as an oral composition. In another embodiment, the compound as defined above is formulated as a long acting parenteral injection and ritonavir is formulated as an injectable composition. In one embodiment, a kit containing the compound as defined above is formulated as a long acting parenteral injection and ritonavir formulated as an injectable composition.

In another embodiment of the present invention, a compound as defined above is used in combination with GS-9350. In one embodiment, the combination is an oral fixed dose combination. In another embodiment, the compound as defined above is formulated as a long acting parenteral injection and GS-9350 is formulated as an oral composition. In one embodiment, there is provided a kit containing the compound as defined above is formulated as a long acting parenteral injection and GS-9350 formulated as an oral composition. In another embodiment, the compound as defined above is formulated as a long acting parenteral injection and GS-9350 is formulated as an injectable composition. In one embodiment, is a kit containing the compound as defined above is formulated as a long acting parenteral injection and GS-9350 formulated as an injectable composition.

In one embodiment of the present invention, a compound as defined above is used in combination with SPI-452. In one embodiment, the combination is an oral fixed dose combination. In another embodiment, the compound of as defined above is formulated as a long acting parenteral injection and SPI-452 is formulated as an oral composition. In one embodiment, there is provided a kit containing the compound as defined above is formulated as a long acting parenteral injection and SPI-452 formulated as an oral composition. In another embodiment, the compound as defined above is formulated as a long acting parenteral injection and SPI-452 is formulated as an injectable composition. In one embodiment, there is provided a kit containing the compound as defined above is formulated as a long acting parenteral injection and SPI-452 formulated as an injectable composition.

In one embodiment of the present invention, a compound as defined above is used in combination with compounds which are found in previously filed PCT/CN2011/0013021.

The above other therapeutic agents, when employed in combination with the chemical entities described herein, may be used, for example, in those amounts indicated in the Physicians' Desk Reference (PDR) or as otherwise determined by one of ordinary skill in the art.

In another embodiment of the invention, there is provided a compound as defined above for use in treating a viral infection in a mammal mediated at least in part by a virus in the *retrovirus* family of viruses wherein said mammal has been diagnosed with said viral infection or is at risk of developing said viral infection.

In another embodiment of the invention, there is provided a compound as defined above for use in treating a viral infection in a mammal mediated at least in part by a virus in the *retrovirus* family of viruses wherein said mammal has been diagnosed with said viral infection or is at risk of developing said viral infection, wherein said virus is an HIV virus. In some embodiments, the HIV virus is the HIV-1 virus.

In another embodiment of the invention, there is provided a compound as defined above and a therapeutically effective amount of one or more agents active against an HIV virus for use in treating a viral infection in a mammal mediated at least in part by a virus in the *retrovirus* family of viruses wherein said mammal has been diagnosed with said viral infection or is at risk of developing said viral infection.

In another embodiment of the invention, there is provided a compound as defined above and a therapeutically effective amount of one or more agents active against the HIV virus for use in treating a viral infection in a mammal mediated at least in part by a virus in the *retrovirus* family of viruses wherein said mammal has been diagnosed with said viral infection or is at risk of developing said viral infection, wherein said agent active against HIV virus is selected from Nucleotide reverse transcriptase inhibitors; Non-nucleotide reverse transcriptase inhibitors; Protease inhibitors; Entry, attachment and fusion inhibitors; Integrase inhibitors; Maturation inhibitors; CXCR4 inhibitors; and CCR5 inhibitors.

In another aspect, the invention provides a compound as defined above or a pharmaceutically acceptable salt thereof for use in depleting latent HIV infected.

In one embodiment of the invention, the invention relates to a compound (compound 1) of the formula: 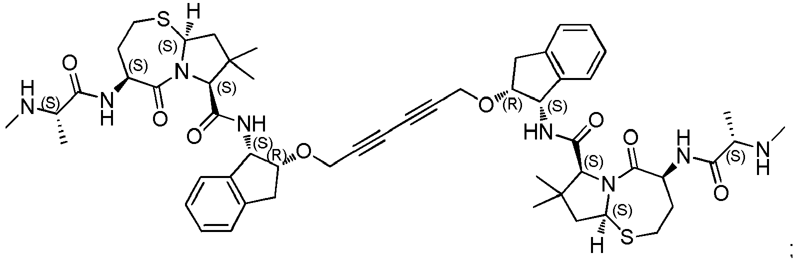 or a pharmaceutically acceptable salt thereof. The invention also includes a pharmaceutical composition comprising this compound, or a pharmaceutically acceptable salt thereof, and a pharmaceutically acceptable excipient including e.g., those set forth herein. The invention also includes this compound or a pharmaceutically acceptable salt thereof, as well as combinations, for use in treating an HIV infection. The invention also includes this compound, or a pharmaceutically acceptable salt thereof, for use in treating an HIV infection. The invention also includes this compound or a pharmaceutically acceptable salt thereof, as well as combinations thereof, for use in depleting latent HIV infected cells.

In various embodiments, depleting latent HIV infection further comprises administering to the subject one or more additional agents active against HIV as disclosed hereinabove. As an example, in various embodiments, the one or more additional agents is selected from the group consisting of nucleotide reverse transcriptase inhibitors, non-nucleotide reverse transcriptase inhibitors, protease inhibitors, entry inhibitors, attachment and fusion inhibitors, integrase inhibitors, maturation inhibitors, CXCR4 and/or CCR5 inhibitors, histone deacetylase inhibitors, histone crotonyl transferase inhibitors, protein kinase C agonists, proteasome inhibitors, TLR7 agonists, bromodomain inbhibitors, and antibodies for clearance therapy, and combinations thereof. In various embodiments, the one or more additional agents active against HIV is selected from the group consisting of zidovudine, didanosine, lamivudine, zalcitabine, abacavir, stavudine, adefovir, adefovir dipivoxil, fozivudine, todoxil, emtricitabine, alovudine, amdoxovir, elvucitabine, nevirapine, delavirdine, efavirenz, loviride, immunocal, oltipraz, capravirine, lersivirine, GSK2248761, TMC-278 TMC-125, etravirine, saquinavir, ritonavir, indinavir, nelfinavir, amprenavir, fosamprenavir, brecanavir, darunavir, atazanavir, tipranavir, palinavir, lasinavir, enfuvirtide, T-20, T-1249, PRO-542, PRO-140, TNX-355, BMS-806, BMS-663068 and BMS-626529, 5-Helix, raltegravir, elvitegravir, dolutegravir,cabotegravir, bictegravir, vicriviroc (Sch-C), Sch-D, TAK779, maraviroc, TAK449, didanosine, tenofovir, lopinavir, darunavir, vorinostat, panobinostat, romidepin, valpronic acid, mocetinostat, sodium corotonate, bryostatin, ingenol B, disulforam, GS-9620, JQ1, iBET151, bortezomib, epigallocatechin gallate, salinosporamide A, carfilzomib, and neutralizing antibodies, eCD4-Ig, CD4-Ig, bNAb, DARTS and IgA.

In another embodiment, there is provided a pharmaceutical composition comprising a pharmaceutically acceptable diluent and a therapeutically effective amount of a compound as defined above or a pharmaceutically acceptable salt thereof.

The compound of the present invention can be supplied in the form of a pharmaceutically acceptable salt. The terms "pharmaceutically acceptable salt" refer to salts prepared from pharmaceutically acceptable inorganic and organic acids and bases. Accordingly, the word "or" in the context of "a compound or a pharmaceutically acceptable salt thereof" is understood to refer to either a compound or a pharmaceutically acceptable salt thereof (alternative), or a compound and a pharmaceutically acceptable salt thereof (in combination).

As used herein, the term "pharmaceutically acceptable" refers to those compounds, materials, compositions, and dosage forms which are, within the scope of sound medical judgment, suitable for use in contact with the tissues of human beings and animals without excessive toxicity, irritation, or other problem or complication. The skilled artisan will appreciate that pharmaceutically acceptable salts of the compound as defined above may be prepared. These pharmaceutically acceptable salts may be prepared *in situ* during the final isolation and purification of the compound, or by separately reacting the purified compound in its free acid or free base form with a suitable base or acid, respectively.

Illustrative pharmaceutically acceptable acid salts of the compound of the present invention can be prepared from the following acids, including, without limitation formic, acetic, propionic, benzoic, succinic, glycolic, gluconic, lactic, maleic, malic, tartaric, citric, nitic, ascorbic, glucuronic, maleic, fumaric, pyruvic, aspartic, glutamic, benzoic, hydrochloric, hydrobromic, hydroiodic, isocitric, trifluoroacetic, pamoic, propionic, anthranilic, mesylic, oxalacetic, oleic, stearic, salicylic, p-hydroxybenzoic, nicotinic, phenylacetic, mandelic, embonic (pamoic), methanesulfonic, phosphoric, phosphonic, ethanesulfonic, benzenesulfonic, pantothenic, toluenesulfonic, 2-hydroxyethanesulfonic, sulfanilic, sulfuric, salicylic, cyclohexylaminosulfonic, algenic, -hydroxybutyric, galactaric and galacturonic acids. Preferred pharmaceutically acceptable salts include the salts of hydrochloric acid and trifluoroacetic acid.

Illustrative pharmaceutically acceptable inorganic base salts of the compound of the present invention include metallic ions. More preferred metallic ions include, but are not limited to, appropriate alkali metal salts, alkaline earth metal salts and other physiological acceptable metal ions. Salts derived from inorganic bases include aluminum, ammonium, calcium, copper, ferric, ferrous, lithium, magnesium, manganic salts, manganous, potassium, sodium, zinc, and the like and in their usual valences. Exemplary base salts include aluminum, calcium, lithium, magnesium, potassium, sodium and zinc. Other exemplary base salts include the ammonium, calcium, magnesium, potassium, and sodium salts. Still other exemplary base salts include, for example, hydroxides, carbonates, hydrides, and alkoxides including NaOH, KOH, Na₂CO₃, K₂CO₃, NaH, and potassium-t-butoxide.

Salts derived from pharmaceutically acceptable organic non-toxic bases include salts of primary, secondary, and tertiary amines, including in part, trimethylamine, diethylamine, N, N'-dibenzylethylenediamine, chloroprocaine, choline, diethanolamine, ethylenediamine, meglumine (N-methylglucamine) and procaine; substituted amines including naturally occurring substituted amines; cyclic amines; quaternary ammonium cations; and basic ion exchange resins, such as arginine, betaine, caffeine, choline, N,N-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

All of the above salts can be prepared by those skilled in the art by conventional means from the corresponding compound of the present invention. For example, the pharmaceutically acceptable salts of the present invention can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, nonaqueous media like ether, ethyl acetate, ethanol, isopropanol, or acetonitrile are preferred. The salt may precipitate from solution and be collected by filtration or may be recovered by evaporation of the solvent. The degree of ionisation in the salt may vary from completely ionised to almost non-ionised. Lists of suitable salts are found in Remington's Pharmaceutical Sciences, 17th ed., Mack Publishing Company, Easton, Pa., 1985, p.1418, as well as in Berge, J. Pharm. Sci., 1977, 66, 1-19, or those listed in P H Stahl and C G Wermuth, editors, Handbook of Pharmaceutical Salts; Properties, Selection and Use, Second Edition Stahl/Wermuth: Wiley-VCH/VHCA, 2011 (see http://www.wiley.com/WileyCDA/ WileyTitle/productCd-3906390519.html.

The compound of the invention may exist in both unsolvated and solvated forms. The term 'solvate' is used herein to describe a molecular complex comprising the compound of the invention and one or more pharmaceutically acceptable solvent molecules, for example, ethanol. The term `hydrate' is employed when said solvent is water. Pharmaceutically acceptable solvates include hydrates and other solvates wherein the solvent of crystallization may be isotopically substituted, *e.g*. D₂O, d₆-acetone, d₆-DMSO.

The compound of the invention contains one or more asymmetric carbon atoms and can exist as two or more stereoisomers. Geometric *cis*/*trans* (or Z/E) isomers are possible. As the compound contains, for example, a keto or oxime group or an aromatic moiety, tautomeric isomerism ('tautomerism') can occur. It follows that the compound may exhibit more than one type of isomerism.

Included within the scope of the claimed compound of the present invention are all stereoisomers, geometric isomers and tautomeric forms of the compound, including compounds exhibiting more than one type of isomerism, and mixtures of one or more thereof. Also included are acid addition or base salts wherein the counterion is optically active, for example, D-lactate or L-lysine, or racemic, for example, DL-tartrate or DL-arginine.

*Cis*/*trans* isomers may be separated by conventional techniques well known to those skilled in the art, for example, chromatography and fractional crystallisation.

Conventional techniques for the preparation/isolation of individual enantiomers include chiral synthesis from a suitable optically pure precursor or resolution of the racemate (or the racemate of a salt or derivative) using, for example, chiral high pressure liquid chromatography (HPLC).

Alternatively, the racemate (or a racemic precursor) may be reacted with a suitable optically active compound, for example, an alcohol, or, in the case where the compound contains an acidic or basic moiety, an acid or base such as tartaric acid or 1-phenylethylamine. The resulting diastereomeric mixture may be separated by chromatography and/or fractional crystallization and one or both of the diastereoisomers converted to the corresponding pure enantiomer(s) by means well known to a skilled person. Chiral compounds of the invention (and chiral precursors thereof) may be obtained in enantiomerically-enriched form using chromatography, typically HPLC, on a resin with an asymmetric stationary phase and with a mobile phase consisting of a hydrocarbon, typically heptane or hexane, containing from 0 to 50% isopropanol, typically from 2 to 20%, and from 0 to 5% of an alkylamine, typically 0.1% diethylamine. Concentration of the eluate affords the enriched mixture.

Mixtures of stereoisomers may be separated by conventional techniques known to those skilled in the art. [see, for example, "Stereochemistry of Organic Compounds" by E L Eliel (Wiley, New York, 1994).]

The present invention includes all pharmaceutically acceptable isotopically-labelled compounds wherein one or more atoms are replaced by atoms having the same atomic number, but an atomic mass or mass number different from the atomic mass or mass number usually found in nature.

Examples of isotopes suitable for inclusion in the compound of the invention include isotopes of hydrogen, such as ²H and ³H, carbon, such as ¹¹C, ¹³C and ¹⁴C, chlorine, such as ³⁶Cl, fluorine, such as ¹⁸F, iodine, such as ¹²³I and ¹²⁵I, nitrogen, such as ¹³N and ¹⁵N, oxygen, such as ¹⁵O, ¹⁷O and ¹⁸O, phosphorus, such as ³²P, and sulphur, such as ³⁵S. Certain isotopically-labelled compounds, for example, those incorporating a radioactive isotope, are useful in drug and/or substrate tissue distribution studies. The radioactive isotopes tritium, *i.e.* ³H, and carbon-14, *i.e.* ¹⁴C, are particularly useful for this purpose in view of their ease of incorporation and ready means of detection.

Substitution with heavier isotopes such as deuterium, *i.e.* ²H, may afford certain therapeutic advantages resulting from greater metabolic stability, for example, increased *in vivo* half-life or reduced dosage requirements, and hence may be preferred in some circumstances.

Isotopically-labelled compounds can generally be prepared by conventional techniques known to those skilled in the art or by processes analogous to those described in the accompanying Examples and Preparations using an appropriate isotopically-labelled reagents in place of the non-labelled reagent previously employed.

The compound of the present invention may be administered as a prodrug. Thus, certain derivatives of the compound of the invention, which may have little or no pharmacological activity themselves can, when administered into or onto the body, be converted into the compound of the invention as a `prodrug'.

Administration of the chemical entities described herein can be *via* any of the accepted modes of administration for agents that serve similar utilities including, but not limited to, orally, sublingually, subcutaneously, intravenously, intranasally, topically, transdermally, intraperitoneally, intramuscularly, intrapulmonarilly, vaginally, rectally, or intraocularly. In some embodiments, oral or parenteral administration is used.

Pharmaceutical compositions or formulations include solid, semi-solid, liquid and aerosol dosage forms, such as, e.g., tablets, capsules, powders, liquids, suspensions, suppositories, aerosols or the like. The chemical entities can also be administered in sustained or controlled release dosage forms, including depot injections, or implant preparation, osmotic pumps, pills, transdermal (including electrotransport) patches, and the like, for prolonged and/or timed, pulsed administration at a predetermined rate. In certain embodiments, the compositions are provided in unit dosage forms suitable for single administration of a precise dose. The active ingredient can be compressed into pellets or small cylinders and implanted subcutaneously or intramuscularly as depot injections or implants. Implants may employ inert materials such as biodegradable polymers or synthetic silicones, for example, Silastic or silicone rubber.

The chemical entities can also be administered in the form of liposome delivery systems, such as small unilamellar vesicles, large unilamellar vesicles and multilamellar vesicles. Liposomes can be formed from a variety of phospholipids, such as cholesterol, stearylamine, or phosphatidylcholines. Liposomal preparations of tyrosine kinase inhibitors may also be used in the methods of the invention. Liposome versions of tyrosine kinase inhibitors may be used to increase tolerance to the inhibitors.

The chemical entities can also be delivered by the use of monoclonal antibodies as individual carriers to which the compound molecules are coupled.

The chemical entities can also be prepared with soluble polymers as targetable drug carriers. Such polymers can include polyvinyl pyrrolidone, pyran copolymer, polyhydroxy-propyl-methacrylamide-phenol, polyhydroxyethyl-aspartamide-phenol, or polyethyleneoxide-polylysine substituted with palmitoyl residues. Furthermore, the chemical entities can be prepared with biodegradable polymers useful in achieving controlled release of a drug, for example, polylactic acid, polyglycolic acid, copolymers of polylactic and polyglycolic acid, polyepsilon caprolactone, polyhydroxy butyric acid, polyorthoesters, polyacetals, polydihydropyrans, polycyanoacrylates and cross linked or amphipathic block copolymers of hydrogels.

The chemical entities described herein can be administered either alone or more typically in combination with a conventional pharmaceutical carrier, excipient or the like (e.g., mannitol, lactose, starch, magnesium stearate, sodium saccharine, talcum, cellulose, sodium crosscarmellose, glucose, gelatin, sucrose, magnesium carbonate, and the like). If desired, the pharmaceutical composition can also contain minor amounts of nontoxic auxiliary substances such as wetting agents, emulsifying agents, solubilizing agents, pH buffering agents and the like (e.g., sodium acetate, sodium citrate, cyclodextrine derivatives, sorbitan monolaurate, triethanolamine acetate, triethanolamine oleate, and the like). Generally, depending on the intended mode of administration, the pharmaceutical composition will contain about 0.005% to 95%; in certain embodiments, about 0.5% to 50% by weight of a chemical entity. Actual methods of preparing such dosage forms are known, or will be apparent, to those skilled in this art; for example, see Remington's Pharmaceutical Sciences, Mack Publishing Company, Easton, Pennsylvania.

In certain embodiments, the compositions will take the form of a pill or tablet and thus the composition will contain, along with the active ingredient, a diluent such as lactose, sucrose, dicalcium phosphate, or the like; a lubricant such as magnesium stearate or the like; and a binder such as starch, gum acacia, polyvinylpyrrolidine, gelatin, cellulose, cellulose derivatives or the like. In another solid dosage form, a powder, marume, solution or suspension (e.g., in propylene carbonate, vegetable oils or triglycerides) is encapsulated in a gelatin capsule.

Liquid pharmaceutically administrable compositions can, for example, be prepared by dissolving, dispersing, etc. at least one chemical entity and optional pharmaceutical adjuvants in a carrier (e.g., water, saline, aqueous dextrose, glycerol, glycols, ethanol or the like) to form a solution or suspension. Injectables can be prepared in conventional forms, either as liquid solutions or suspensions, as emulsions, or in solid forms suitable for dissolution or suspension in liquid prior to injection. The percentage of chemical entities contained in such parenteral compositions is highly dependent on the specific nature thereof, as well as the activity of the chemical entities and the needs of the subject. However, percentages of active ingredient of 0.01% to 10% in solution are employable, and will be higher if the composition is a solid which will be subsequently diluted to the above percentages. In certain embodiments, the composition will comprise from about 0.2 to 2% of the active agent in solution.

Pharmaceutical compositions of the chemical entities described herein may also be administered to the respiratory tract as an aerosol or solution for a nebulizer, or as a microfine powder for insufflation, alone or in combination with an inert carrier such as lactose. In such a case, the particles of the pharmaceutical composition have diameters of less than 50 microns, in certain embodiments, less than 10 microns.

In general, the chemical entities provided will be administered in a therapeutically effective amount by any of the accepted modes of administration for agents that serve similar utilities. The dosage regimen utilizing the chemical entities described herein can be selected in accordance with a variety of factors including type, species, age, weight, sex and the type of disease being treated; the severity (i.e., stage) of the disease to be treated; the route of administration; the renal and hepatic function of the patient; and the particular compound or salt thereof employed. A dosage regimen can be used, for example, to prevent, inhibit (fully or partially), or arrest the progress of the disease.The drug can be administered more than once a day, such as once or twice a day.

Intravenously or subcutaneously, the patient would receive the chemical entities described herein in therapeutically effective amounts sufficient to deliver between about 0.001 to 200 mg per kilogram body weight of the recipient per day; such as about 0.005-100 mg/kg/day, for example, from about 0.005 to 1 mg/kg/day. Thus, for administration to a 70 kg person, the dosage range may be about 0.35-70 mg per day. Such quantities may be administered in a number of suitable ways, e.g. large volumes of low concentrations of the chemical entities during one extended period of time or several times a day. The quantities can be administered for one or more consecutive days, intermittent days or a combination thereof per week (7-day period). Alternatively, low volumes of high concentrations of the chemical entities during a short period of time, e.g. once a day for one or more days either consecutively, intermittently or a combination thereof per week (7-day period).

In accordance with the invention, the chemical entities described herein can be administered by continuous or intermittent dosages. For example, intermittent administration of the chemical entity may be administration one to six days per week or it may mean administration in cycles (e.g. daily administration for two to eight consecutive weeks, then a rest period with no administration for up to one week) or it may mean administration on alternate days. The compositions may be administered in cycles, with rest periods in between the cycles (e.g. treatment for two to eight weeks with a rest period of up to a week between treatments).

Subcutaneous formulations can be prepared according to procedures well known in the art at a pH in the range between about 5 and about 12, which include suitable buffers and isotonicity agents.

In general, the chemical entities will be administered as pharmaceutical compositions by any one of the following routes: oral, systemic (e.g., transdermal, intranasal or by suppository), or parenteral (e.g., intramuscular, intravenous or subcutaneous) administration. In certain embodiments, oral administration with a convenient daily dosage regimen that can be adjusted according to the degree of affliction may be used. Compositions can take the form of tablets, pills, capsules, semisolids, powders, sustained release formulations, solutions, suspensions, elixirs, aerosols, or any other appropriate compositions. Another manner for administering the provided chemical entities is inhalation.

The choice of formulation depends on various factors such as the mode of drug administration and bioavailability of the drug substance. For delivery *via* inhalation the chemical entity can be formulated as liquid solution, suspensions, aerosol propellants or dry powder and loaded into a suitable dispenser for administration. There are several types of pharmaceutical inhalation devices-nebulizer inhalers, metered dose inhalers (MDI) and dry powder inhalers (DPI). Nebulizer devices produce a stream of high velocity air that causes the therapeutic agents (which are formulated in a liquid form) to spray as a mist that is carried into the patient's respiratory tract. MDIs typically are formulation packaged with a compressed gas. Upon actuation, the device discharges a measured amount of therapeutic agent by compressed gas, thus affording a reliable method of administering a set amount of agent. DPI dispenses therapeutic agents in the form of a free flowing powder that can be dispersed in the patient's inspiratory air-stream during breathing by the device. In order to achieve a free flowing powder, the therapeutic agent is formulated with an excipient such as lactose. A measured amount of the therapeutic agent is stored in a capsule form and is dispensed with each actuation.

Recently, pharmaceutical compositions have been developed for drugs that show poor bioavailability based upon the principle that bioavailability can be increased by increasing the surface area i.e., decreasing particle size. For example, U.S. Patent No. 4,107,288 describes a pharmaceutical formulation having particles in the size range from 10 to 1,000 nm in which the active material is supported on a cross-linked matrix of macromolecules. U.S. Patent No. 5,145,684 describes the production of a pharmaceutical formulation in which the drug substance is pulverized to nanoparticles (average particle size of 400 nm) in the presence of a surface modifier and then dispersed in a liquid medium to give a pharmaceutical formulation that exhibits remarkably high bioavailability.

The compositions are comprised of, in general, at least one chemical entity described herein in combination with at least one pharmaceutically acceptable excipient. Acceptable excipients are non-toxic, aid administration, and do not adversely affect the therapeutic benefit of the at least one chemical entity described herein. Such excipient may be any solid, liquid, semi-solid or, in the case of an aerosol composition, gaseous excipient that is generally available to one of skill in the art.

Solid pharmaceutical excipients include starch, cellulose, talc, glucose, lactose, sucrose, gelatin, malt, rice, flour, chalk, silica gel, magnesium stearate, sodium stearate, glycerol monostearate, sodium chloride, dried skim milk and the like. Liquid and semisolid excipients may be selected from glycerol, propylene glycol, water, ethanol and various oils, including those of petroleum, animal, vegetable or synthetic origin, e.g., peanut oil, soybean oil, mineral oil, sesame oil, etc. Liquid carriers, for injectable solutions, include water, saline, aqueous dextrose, and glycols.

Compressed gases may be used to disperse a chemical entity described herein in aerosol form. Inert gases suitable for this purpose are nitrogen, carbon dioxide, etc. Other suitable pharmaceutical excipients and their formulations are described in Remington's Pharmaceutical Sciences, edited by E. W. Martin (Mack Publishing Company, 18th ed., 1990).

The amount of the chemical entity in a composition can vary within the full range employed by those skilled in the art. Typically, the composition will contain, on a weight percent (wt%) basis, from about 0.01-99.99 wt% of at least one chemical entity described herein based on the total composition, with the balance being one or more suitable pharmaceutical excipients. In certain embodiments, the at least one chemical entity described herein is present at a level of about 1-80 wt%.

In various embodiments, pharmaceutical compositions of the present invention encompass the compound of the invention, salts thereof, and combinations of the above.

The various modes of administration, dosages, and dosing schedules described herein merely set forth specific embodiments and should not be construed as limiting the broad scope of the invention. Any permutations, variations, and combinations of the dosages and dosing schedules are included within the scope of the present invention.

### Synthetic Methods

The methods of synthesis for the provided chemical entities employ readily available starting materials using the following general methods and procedures. It will be appreciated that where typical or preferred process conditions (*i.e.*, reaction temperatures, times, mole ratios of reactants, solvents, pressures, etc.) are given; other process conditions can also be used unless otherwise stated. Optimum reaction conditions may vary with the particular reactants or solvent used, but such conditions can be determined by one skilled in the art by routine optimization procedures.

Additionally, the methods of this invention may employ protecting groups which prevent certain functional groups from undergoing undesired reactions. Suitable protecting groups for various functional groups as well as suitable conditions for protecting and deprotecting particular functional groups are well known in the art. For example, numerous protecting groups are described in T. W. Greene and G. M. Wuts, Protecting Groups in Organic Synthesis, Third Edition, Wiley, New York, 1999, and references cited therein.

Furthermore, the provided chemical entities may contain one or more chiral centers and such compounds can be prepared or isolated as pure stereoisomers, *i.e.,* as individual enantiomers or diastereomers, or as stereoisomer-enriched mixtures. All such stereoisomers (and enriched mixtures) are included within the scope of this specification, unless otherwise indicated. Pure stereoisomers (or enriched mixtures) may be prepared using, for example, optically active starting materials or stereoselective reagents well-known in the art. Alternatively, racemic mixtures of such compounds can be separated using, for example, chiral column chromatography, chiral resolving agents and the like.

The starting materials for the following reactions are generally known compounds or can be prepared by known procedures or obvious modifications thereof. For example, many of the starting materials are available from commercial suppliers such as Aldrich Chemical Co. (Milwaukee, Wisconsin, USA), Bachem (Torrance, California, USA), Ernka-Chemce or Sigma (St. Louis, Missouri, USA). Others may be prepared by procedures, or obvious modifications thereof, described in standard reference texts such as Fieser and Fieser's Reagents for Organic Synthesis, Volumes 1-15 (John Wiley and Sons, 1991), Rodd's Chemistry of Carbon Compounds, Volumes 1-5 and Supplementals (Elsevier Science Publishers, 1989), Organic Reactions, Volumes 1-40 (John Wiley and Sons, 1991), March's Advanced Organic Chemistry, (John Wiley and Sons, 4th Edition), and Larock's Comprehensive Organic Transformations (VCH Publishers Inc., 1989).

Unless specified to the contrary, the reactions described herein take place at atmospheric pressure, generally within a temperature range from -78 C to 200 C. Further, except as employed in the Examples or as otherwise specified, reaction times and conditions are intended to be approximate, e.g., taking place at about atmospheric pressure within a temperature range of about -78 C to about 110 C over a period of about 1 to about 24 hours; reactions left to run overnight average a period of about 16 hours.

The terms "solvent," "organic solvent," and "inert solvent" each mean a solvent inert under the conditions of the reaction being described in conjunction therewith, including, for example, benzene, toluene, acetonitrile, tetrahydrofuranyl ("THF"), dimethylformamide ("DMF"), chloroform, methylene chloride (or dichloromethane), diethyl ether, methanol, N-methylpyrrolidone ("NMP"), pyridine and the like.

Isolation and purification of the chemical entities and intermediates described herein can be effected, if desired, by any suitable separation or purification procedure such as, for example, filtration, extraction, crystallization, column chromatography, thin-layer chromatography or thick-layer chromatography, or a combination of these procedures. Specific illustrations of suitable separation and isolation procedures can be had by reference to the examples herein below. However, other equivalent separation or isolation procedures can also be used.

When desired, the (R)- and (S)-isomers may be resolved by methods known to those skilled in the art, for example by formation of diastereoisomeric salts or complexes which may be separated, for example, by crystallization; *via* formation of diastereoisomeric derivatives which may be separated, for example, by crystallization, gas-liquid or liquid chromatography; selective reaction of one enantiomer with an enantiomer-specific reagent, for example enzymatic oxidation or reduction, followed by separation of the modified and unmodified enantiomers; or gas-liquid or liquid chromatography in a chiral environment, for example on a chiral support, such as silica with a bound chiral ligand or in the presence of a chiral solvent. Alternatively, a specific enantiomer may be synthesized by asymmetric synthesis using optically active reagents, substrates, catalysts or solvents, or by converting one enantiomer to the other by asymmetric transformation.

### EXAMPLES

The following examples serve to more fully describe the manner of making and using the above-described invention. In the examples below, the following abbreviations have the following meanings. If an abbreviation is not defined, it has its generally accepted meaning.
- aq.: = Aqueous
- µL: = Microliters
- µM: = Micromolar
- NMR: = nuclear magnetic resonance
- Boc: = tert-butoxycarbonyl
- Br: = Broad
- Cbz: = Benzyloxycarbonyl
- d: = Doublet
- Δ: = chemical shift
- °C: = degrees celcius
- DCM: = Dichloromethane
- dd: = doublet of doublets
- DMAP: = 4-(Dimethylamino)pyridine
- DMEM: = Dulbeco's Modified Eagle's Medium
- DMF: = N,N-dimethylformamide
- DMP: = 2,2-dimethoxypropane
- DMSO: = Dimethylsulfoxide
- EtOAc: = ethyl acetate
- ESI: = electrospray ionization
- G or g: = Gram
- h or hr: = Hours
- HCV: = hepatitus C virus
- HPLC: = high performance liquid chromatography
- Hz: = Hertz
- IU: = International Units
- IC₅₀: = inhibitory concentration at 50% inhibition
- J: = coupling constant (given in Hz unless otherwise indicated)
- LHMDS: = Lithium bis(trimethylsilyl)amide
- M: = Multiplet
- M: = Molar
- M+H⁺: = parent mass spectrum peak plus H⁺
- Mg or mg: = Milligram
- Min: = Minutes
- mL: = Milliliter
- mM: = Millimolar
- Mmol: = Millimole
- MS: = mass spectrum
- Nm: = Nanomolar
- ppm: = parts per million
- p-TsOH: = p-Toluenesulfonic acid
- q.s.: = sufficient amount
- S: = Singlet
- RT: = room temperature
- sat.: = Saturated
- T: = Triplet
- TBS-CI: = tert-Butyldimethylsilyl chloride
- TFA: = trifluoroacetic acid

### Equipment Description

¹H NMR spectra were recorded on a Varian spectrometer. Chemical shifts are expressed in parts per million (ppm, units). Coupling constants are in units of hertz (Hz). Splitting patterns describe apparent multiplicities and are designated as s (singlet), d (doublet), t (triplet), q (quartet), quint (quintet), m (multiplet), br (broad).

The analytical low-resolution mass spectra (MS) were recorded on Waters (Acquity). The following conditions were employed described below.
Instrument: Agilent 1200-6100
Scan Mode: Alternating Positive/Negative Electrospray
Scan Range: 100-1000 amu

### LC Conditions:

The LCMS analysis was conducted on a HALO C-18, 4.6*50 mm, 2.7 µm, C18 column at 45 °C.

1.0 uL of sample was injected

The gradient employed was:
Mobile Phase A: Water + 0.1 % v/v Formic Acid
Mobile Phase B: Acetonitrile + 0.1 % v/v Formic Acid

| **Time** | **%A** | **%B** | **Flow Rate** |
|---|---|---|---|
| 0.00 min | 95 | 5 | 1.8 ml/min |
| 1.0 min | 5 | 95 | 1.8 ml/min |
| 2.0 min | 5 | 95 | 1.8 ml/min |
| 2.5 min | 95 | 5 | 1.8 ml/min |

### UV detection provided by summed absorbance signal at 214 nm and 254 nm scanning

Instrument: Shimadzu LCMS-2020
Scan Mode: Alternating Positive/Negative Electrospray
Scan Range: 100-2000 amu

### LC Conditions:

The LCMS analysis was conducted on a HALO C-18, 4.6*50 mm, 2.7 µm, C18 column at 45 ⁰C.

1.0 uL of sample was injected

The gradient employed was:
Mobile Phase A: Water + 0.1 % v/v Formic Acid
Mobile Phase B: Acetonitrile + 0.1 % v/v Formic Acid

| **Time** | **%A** | **%B** | **Flow Rate** |
|---|---|---|---|
| 0.00 min | 95 | 5 | 1.5 ml/min |
| 1.0 min | 5 | 95 | 1.5 ml/min |
| 2.0 min | 5 | 95 | 1.5 ml/min |
| 2.5 min | 95 | 5 | 1.5 ml/min |
| 3.0 min | 95 | 5 | 1.5 ml/min |

UV detection provided by summed absorbance signal at 214 nm and 254 nm scanning

### Intermediate I: (4S,7S,9aS)-4-((S)-2-((tert-butoxycarbonyl)(methyl)amino)propanamido)-8,8-dimethyl-5-oxooctahydropyrrolo[2,1-b][1,3]thiazepine-7-carboxylic acid

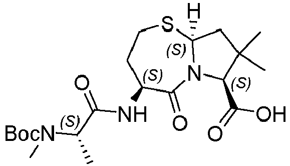

### Step 1: (E)-1-(2-nitrostyryl)pyrrolidine

To a solution of 1-methyl-2-nitrobenzene (50 g, 365mmol) in *N,N*-dimethylformamide (200 mL) was added pyrrolidine (31.1 g, 438 mmol) and 1,1-dimethoxy-*N*,*N-*dimethylmethanamine (52.2 g, 438 mmol). The resulting mixture was stirred at 60 °C. After 5 h, the temperature was increased to 80 °C and stirred for 17 h. Upon cooling to room temperature, the mixture was partitioned between methyl tert-butyl ether (500 mL) and water (1 L). The aqueous phase was separated and extracted with methyl tert-butyl ether (500 mL). The combined organic layers were washed with brine,dried over anhydrous sodium sulfate, filtered and concentrated to give (E)-1-(2-nitrostyryl)pyrrolidine (80 g, crude) as dark red oil. This was used for the next step without further purification.

### Step 2: 1-(2,2-dimethoxyethyl)-2-nitrobenzene

To a solution of (*E*)-1-(2-nitrostyryl)pyrrolidine (80 g, crude) in methanol (600 mL) was added trimethylchlorosilane (59.5 g, 550 mmol) slowly. The mixture was heated to reflux for 24 h. At which time the solution was allowed to cool to room temperature and concentrated in vacuo to get the residue. It was partitioned between ethyl acetate (500 mL) and 5% aqueous citric acid (800 mL). The aqueous layer was extracted with ethyl acetate (2 × 300 mL). The combined organic layers were washed with 5 % aqueous sodium bicarbonate (300 mL) followed by brine. The crude was dried over anhydrous sodium sulfate, filtered and concentrated to give 1-(2,2-dimethoxyethyl)-2-nitrobenzene (79 g, crude) as dark red oil which was used for the next step without further purification.

### Step 3: 2-(2,2-dimethoxyethyl)aniline

To a solution of 1-(2,2-dimethoxyethyl)-2-nitrobenzene (79 g, 374.4 mmol) in methanol (1 L) was added palladium on activated carbon (17.6 g). The mixture was stirred under hydrogen at 50 psi at room temperature for 17 h. The mixture was filtered through diatomite and rinsed with methanol. The filtrate was concentrated to give the crude product. The crude was dissolved in methyl tert-butyl ether (500 mL) and filtered. The filtrate was concentrated to give 2-(2,2-dimethoxyethyl)aniline (60 g, 331.5 mmol, 88.5% yield) as dark red oil. This was used for the next step without further purification. ¹H NMR (400 MHz, DMSO-*d*₆) δ ppm 6.98 - 6.89 (m, 2H), 6.63 (dd, *J* = 7.9, 1.1 Hz, 1H), 6.51 (td, *J* = 7.4, 1.2 Hz, 1H), 4.80 (s, 2H), 4.55 (t, *J=* 5.6 Hz, 1H), 3.25 (s, 6H), 2.72 (d, *J=* 5.6 Hz, 2H).

### Step 4: N-(2-(2,2-dimethoxyethyl)phenyl)formamide

To a solution of 2-(2,2-dimethoxyethyl)aniline (60 g, 331.5 mmol) and ethyl formate (36.8 g, 497.2 mmol) in dry tetrahydrofuran (400 mL) was added a solution of 1 M lithium bis(trimethylsilyl)amide (597 mL, 597 mmol) slowly. The mixture was stirred at room temperature for 12 h and then refluxed for 18 h. At which time saturated ammonium chloride (200 mL) was added and the mixture was extracted with ethyl acetate (3 × 200 mL). The organic phases were combined, dried over anhydrous sodium sulfate, filtered and concentrated. The crude was purified by silica gel chromatography [petroleum ether/ethyl acetate (6:1 v/v)] to afford *N*-(2-(2,2 dimethoxyethyl)phenyl)formamide (58 g, 277.5 mmol, 83.7% yield) as dark red oil.

LCMS (2.5 min formic acid): Rt = 1.354 min, m/z: 209.1 [M+1]⁺, 231.9 [M+Na]⁺.

### Step 5: 1-(2,2-dimethoxyethyl)-2-isocyanobenzene

To a solution of *N*-(2-(2,2 dimethoxyethyl)phenyl)formamide (58 g, 277.5 mmol) in dichloromethane (500 mL) at 0 °C was added triethylamine (143 g, 1415.3 mmol) followed by phosphorus oxychloride (63.9 g, 416.3 mmol). The mixture was warmed to room temperature and stirred for 2 h. At which time it was poured into saturated aqueous sodium bicarbonate (300 mL) and extracted with dichloromethane (3 × 200 mL). The organic layers were dried over anhydrous sodium sulfate, filtered and concentrated. The crude was purified by silica gel chromatography [petroleum ether/ethyl acetate (20:1 v/v)] to afford 1-(2,2-dimethoxyethyl)-2-isocyanobenzene (42 g, 218.5 mmol, 79.2% yield) as a pale brown oil. ¹H NMR (400 MHz, DMSO-*d*₆) δ ppm 7.57 - 7.27 (m, 4H), 4.61 (t, *J* = 5.6 Hz, 1H), 3.27 (s, 6H), 3.00 (d, *J=* 5.6 Hz, 2H). LCMS (2.5 min formic acid): Rt = 1.455 min, m/z: 192.0 (M+1)⁺.

### Step 6: 4,4-dimethoxy-2,2-dimethylbutanenitrile

To a solution of diisopropylamine (89.4 mL, 682 mmol) in tetrahydrofuran (1 L) at -10 °C under nitrogen was added a solution of 2.4 M n-butyl lithium in hexane (288 mL, 682 mmol). After 30 min, the mixture was cooled to -78 °C and a solution of 4,4-dimethoxybutanenitrile (40 g, 310 mmol) in tetrahydrofuran (30 mL) was added. After 1 h methyl iodide (42.4 mL, 682 mmol) was added very slowly. The mixture was allowed to warm to room temperature and stirred overnight. At which time it was quenched with saturated aqueous ammonium chloride and extracted with ethyl acetate (3 × 400 mL). The organic layers were dried over anhydrous sodium sulfate, filtered and concentrated. The crude was purified by silica gel chromatography [petroleum ether/ethyl acetate (30:1 v/v)] to afford 4,4-dimethoxy-2,2-dimethylbutanenitrile (35 g, 223 mmol, 71.9% yield) as pale yellow oil. ¹H-NMR (400 MHz, CDCl₃) δ ppm 4.60 (t, *J=* 5.4 Hz, 1H), 3.36 (s, 6H), 1.83 (d, *J=* 5.4 Hz, 2H), 1.39 (s, 6H).

### Step 7: 4,4-dimethoxy-2,2-dimethylbutanal

To a solution of 4,4-dimethoxy-2,2-dimethylbutanenitrile (27 g, 172 mmol) in dichloromethane (800 mL) was added a solution of 1 M diisobutylaluminium hydride in hexane (189 mL, 189 mmol) slowly at -78 °C for 3.5 h. At which time it was warmed to room temperature and quenched with saturated aqueous ammonium chloride (400 mL) and Rochelle salt (400 mL). The aqueous phase was extracted with dichloromethane (2 × 400 mL). The combined organic layers were washed with brine (400 mL), dried over anhydrous sodium sulfate, filtered and concentrated. The crude was purified by silica gel chromatography [petroleum ether/ethyl acetate (30:1 v/v)] to afford 4,4-dimethoxy-2,2-dimethylbutanal (13 g, 81.1 mmol, 47.1% yield) as a colorless oil. LCMS (2.5 min formic acid): Rt = 1.453 min, m/z: 182.9 (M+Na)⁺.

### Step 8: (4S,9aS)-4-amino-7-(1H-indole-1-carbonyl)-8,8-dimethylhexahydropyrrolof2,1-b][1,3]thiazepin-5(2H)-one

a. A mixture of *N*-(*tert*-butoxycarbonyl)-*S*-trityl-*L*-homocysteine (11.8 g, 24.7 mmol), 1-(2,2-dimethoxyethyl)-2-isocyanobenzene (from Step 5) (4.5 g, 23.5 mmol), 4,4-dimethoxy-2,2-dimethylbutanal (from Step 7) (4.5 g, 28.2 mmol) and 7 M ammonia in methanol (10 mL) in 2,2,2-trifluoroethanol (150 mL) was stirred at 100 °C for 2 h. At which time it was quenched with 1 M aq. sodium hydroxide solution (200 mL) and extracted with ethyl acetate (3 × 150 mL). The combined organic phases were dried over anhydrous sodium sulfate, filtered and concentrated to give crude *tert*-butyl ((2*S*)-1-((1-((2-(2,2-dimethoxyethyl)phenyl)amino)-5,5-dimethoxy-3,3-dimethyl-1-oxopentan-2-yl)amino)-1-oxo-4-(tritylthio)butan-2-yl)carbamate (26 g, crude) as brown solid. It was used for the next step without any further purification.
b. Further to step a, to a solution of crude *tert*-butyl ((2*S*)-1-((1-((2-(2,2-dimethoxyethyl)phenyl)amino)-5,5-dimethoxy-3,3-dimethyl-1-oxopentan-2-yl)amino)-1-oxo-4-(tritylthio)butan-2-yl)carbamate (26 g, crude) in dichloromethane (200 mL) was added trifluoroacetic acid (20 mL) slowly. The mixture was stirred at 40 °C for 2 h. The solvent was concentrated and the crude was purified by silica gel chromatography [dichloromethane / methanol (25:1 v/v)] to afford (4*S*,9a*S*)-4-amino-7-(1H-indole-1-carbonyl)-8,8-dimethylhexahydropyrrolo[2,1-b][1,3]thiazepin-5(2H)-one (7.5 g, 21.0 mmol) as a sandy solid. LCMS (2.5 min formic acid): Rt = 1.361 min, m/z: 357.9 (M+1)⁺.

### Step 9: tert-butyl ((S)-1-(((4S,7S,9aS)-7-(1H-indole-1-carbonyl)-8,8-dimethyl-5-oxooctahydropyrrolo[2,1-b1[1,3]thiazepin-4-yl)amino)-1-oxopropan-2-yl)(methyl)carbamate

To a solution of (4S,9aS)-4-amino-7-(1H-indole-1-carbonyl)-8,8-dimethylhexahydropyrrolo[2,1-b][1,3]thiazepin-5(2H)-one (14 g, 39.2 mmol), *N-*(*tert-*butoxycarbonyl)-*N*-methyl*-L*-alanine (8.76 g, 43.1 mmol), 1-hydroxybenzotriazole (5.82 g, 43.1 mmol) and 4-methylmorpholine (11.88 g, 117.6 mmol) in tetrahydrofuran (400 mL) was added *N*-(3-dimethylaminopropyl)-*N*'-ethylcarbodiimide hydrochloride (8.27 g, 43.1 mmol). The mixture was stirred at room temperature for 3 h. At which time it was quenched with saturated aqueous sodium bicarbonate solution (300 mL) and extracted with ethyl acetate (3 × 150 mL). The combined organic layers were washed with brine (200 mL), dried over anhydrous sodium sulfate, filtered and concentrated. The crude was purified by silica gel chromatography [petroleum ether/ethyl acetate (2:1 v/v to 1:1 v/v)] to afford tert-butyl ((S)-1-(((4S,7R,9aS)-7-(1H-indole-1-carbonyl)-8,8-dimethyl-5-oxooctahydropyrrolo[2,1-b][1,3]thiazepin-4-yl)amino)-1-oxopropan-2-yl)(methyl)carbamate (5.2 g, 9.6 mmol, 24.5 % yield) as sandy solid and the desired product *tert*-butyl ((*S*)-1-(((4*S*,7*S*,9a*S*)-7-(1H-indole-1-carbonyl)-8,8-dimethyl-5-oxooctahydropyrrolo[2,1-b][1,3]thiazepin-4-yl)amino)-1-oxopropan-2-yl)(methyl)carbamate (5.0 g, 9.2 mmol, 23.5% yield) as pale yellow solid. LCMS (2.5 min formic acid): Rt = 1.740 min, m/z: 564.8 (M+1)⁺.

### Step 10: (4S,7S,9aS)-4-((S)-2-((tert-butoxycarbonyl)(methyl)amino)propanamido)-8,8-dimethyl-5-oxooctahydropyrrolo[2,1-b][1,3]thiazepine-7-carboxylic acid

To a solution of *tert-*butyl ((*S*)-1-(((4*S*,7*S*,9a*S*)-7-(1H-indole-1-carbonyl)-8,8-dimethyl-5-oxooctahydropyrrolo[2,1-b][1,3]thiazepin-4-yl)amino)-1-oxopropan-2-yl)(methyl)carbamate (3.9 g, 7.19 mmol) in methanol (60 mL) was added 1 M aqueous sodium hydroxide solution (60 mL). The mixture was stirred at room temperature for 24 h. The volatile solvent was removed under reduced pressure. The remaining aqueous phase was washed with ethyl acetate (50 mL) and the pH of the aqueous phase was adjusted with citric acid to pH 3. The aqueous phase was extracted with ethyl acetate (3 × 60 mL). The combined organic phases were washed with brine (100 mL), dried over anhydrous sodium sulfate, filtered and concentrated. The crude was purified by silica gel chromatography [petroleum ether/ethyl acetate (1:2 v/v)] to afford (4*S*,7*S*,9a*S*)-4-((*S*)-2-((*tert-*butoxycarbonyl)(methyl)amino)propanamido)-8,8-dimethyl-5-oxooctahydropyrrolo[2,1-b][1,3]thiazepine-7-carboxylic acid (2 g, 4.51 mmol, 62.7% yield) as a white solid. LCMS (2.5 min formic acid): Rt = 1.493 min, m/z: 443.9 (M+1). ¹*H NMR (400 MHz,* CDC*l*₃) *δ ppm 12.57 (s, 1H), 7.75 (d, J = 6.6 Hz, 1H), 5.46 (t, J = 7.2 Hz, 1H), 4.64* - *4.60 (m, 1H), 4.43 (s, 1H), 3.98 (s, 1H), 3.17* - *3.11 (m, 1H), 2.88 (dd, J = 14.6, 3.0 Hz, 1H), 2.74 (s, 3H), 2.27 (dd, J = 12.9, 7.5 Hz, 1H), 2.12* - *2.10 (m, 1H), 1.80 (dd, J = 13.0, 7.1 Hz, 1H), 1.74- 1.68 (m, J = 11.2 Hz, 1H), 1.40 (s, 9H), 1.25 (d, J = 7.1 Hz, 3H), 1.09 (d, J = 10.6 Hz, 6H).*

### Example 1

### (4S,4'S,7S,7'S,9aS,9a'S)-N,N'-((1S,1'S,2R,2'R)-(hexa-2,4-diyne-1,6-diylbis(oxy))bis(2,3-dihydro-1H-indene-2,1-diyl))bis(8,8-dimethyl-4-((S)-2-(methylamino)propanamido)-5-oxooctahydropyrrolo[2,1-b][1,3]thiazepine-7-carboxamide) dihydrochloride

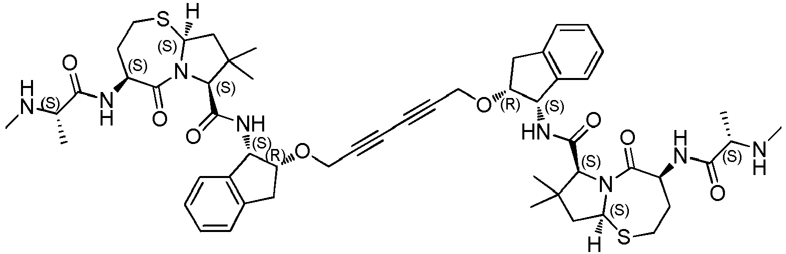

### Step 1: (1S,2R)-2-(prop-2-yn-1-yloxy)-2,3-dihydro-1H-inden-1-amine

To a solution of (1S,2R)-1-amino-2,3-dihydro-1H-inden-2-ol (2 g, 13.4 mmol) in tetrahydrofuran (50 mL) at 0 °C was added portion wise sodium hydride (60%, dispersion in Paraffin Liquid) (0.59 g, 14.7 mmol). The mixture was allowed to warm to room temperature. 3-Bromoprop-1-yne (1.75 g, 14.7 mmol) was then added and the resulting mixture was heated to 70 °C. It was stirred at 70 °C overnight. The mixture was quenched with ice water (50 mL) and extracted with ethyl acetate (3 × 30 mL). The combined organic layers were washed with brine (50 mL), dried over anhydrous sodium sulfate, filtered and concentrated. The crude was purified by silica gel chromatography [petroleum ether/ethyl acetate (3:2 v/v)] to afford (1S,2R)-2-(prop-2-yn-1-yloxy)-2,3-dihydro-1H-inden-1-amine (1.2 g, 6.4 mmol, 47.8 % yield) as black oil. *LCMS (ES, m*/*z): 187.1,* 188.1 *[M+H]⁺, retention time 0.942 min.* ¹H NMR (400 MHz, DMSO-*d*₆) δ ppm 7.36 - 7.29 (m, 1H), 7.22 - 7.13 (m, 3H), 4.25 (dd, *J* = 4.2, 2.4 Hz, 2H), 4.22 - 4.15 (m, 2H), 3.43 (t, *J=* 2.4 Hz, 1H), 2.93 (qd, J= 16.1, 3.5 Hz, 2H), 1.75 (s, 2H).

### Step 2: tert-butyl ((S)-1-(((4S,7S,9aS)-8,8-dimethyl-5-oxo-7-(((1S,2R)-2-(prop-2-yn-1-yloxy)-2,3-dihydro-1H-inden-1-yl)carbamoyl)octahydropyrrolo[2,1-bir1,3]thiazepin-4-yl)amino)-1-oxopropan-2-yl)(methyl)carbamate

To a solution of (4S,7S,9aS)-4-((S)-2-((tert-butoxycarbonyl)(methyl)amino)propanamido)-8,8-dimethyl-5-oxooctahydropyrrolo[2,1-b][1,3]thiazepine-7-carboxylic acid (260 mg, 0.587 mmol), 2-(7-azabenzotriazol-1-yl)-N,N,N',N'-tetramethyluronium hexafluorophosphate (305 mg, 0.801 mmol) and N,N-diisopropylethylamine (138 mg, 1.068 mmol) in 1,2-dichloroethane (10 mL) was added (1S,2R)-2-(prop-2-yn-1-yloxy)-2,3-dihydro-1H-inden-1-amine (100 mg, 0.534 mmol). The mixture was stirred at 50 °C overnight. The solvent was removed under reduced pressure. The crude was purified by silica gel chromatography [petroleum ether/ethyl acetate (1:1 v/v)] and further purified by thin layer chromatography [ethyl acetate/ petroleum ether (3/2 v/v)] to give tert-butyl ((S)-1-(((4S,7S,9aS)-8,8-dimethyl-5-oxo-7-(((1S,2R)-2-(prop-2-yn-1-yloxy)-2,3-dihydro-1H-inden-1-yl)carbamoyl)octahydropyrrolo[2,1-b][1,3]thiazepin-4-yl)amino)-1-oxopropan-2-yl)(methyl)carbamate (160 mg, 0.261 mmol, 48.9 % yield). LCMS (2.5 min formic acid): Rt = 1.662 min, m/z: 634.8 (M+Na)⁺.

### Step 3: di-tert-butyl ((2S,2'S)-(((4S,4'S,7S,7'S,9aS,9a'S)-((((1S,1'S,2R,2'R)-(hexa-2,4-diyne-1,6-diylbis(oxy))bis(2,3-dihydro-1H-indene-2,1-diyl))bis(azanediyl))bis(carbonyl))bis(8,8-dimethyl-5-oxooctahydropyrrolo[2,1-b][1,31thiazepine-7,4-diyl))bis(azanediyl))bis(1-oxopropane-1,2-diyl))bis(methylcarbamate)

a. To a solution of tert-butyl ((S)-1-(((4S,7S,9aS)-8,8-dimethyl-5-oxo-7-(((1S,2R)-2- (prop-2-yn-1-yloxy)-2,3-dihydro-1H-inden-1-yl)carbamoyl)octahydropyrrolo[2,1-b][1,3]thiazepin-4-yl)amino)-1-oxopropan-2-yl)(methyl)carbamate (160 mg, 0.261 mmol) and pyridine (124 mg, 1.566 mmol) in acetonitrile (7 mL) was added cupric acetate (57 mg, 0.313 mmol). The mixture was stirred at 85 °C for 1h. The mixture was cooled, concentrated and diluted with ethyl acetate (15 mL). Aqueous ammonia (20 fold dilution,15 mL) was added. The aqueous phase was extracted with ethyl acetate (2 × 10 mL). The combined organic layers were washed with brine (15 mL), dried over anhydrous sodium sulfate, filtered and concentrated. The crude was purified by thin layer chromatography [ethyl acetate / petroleum ether (2/1 v.v)] followed by Prep-HPLC to give di-tert-butyl ((2S,2'S)-(((4S,4'S,7S,7'S,9aS,9a'S)-((((1S,1'S,2R,2'R)-(hexa-2,4-diyne-1,6-diylbis(oxy))bis(2,3-dihydro-1H-indene-2,1-diyl))bis(azanediyl))bis(carbonyl))bis(8,8-dimethyl-5-oxooctahydropyrrolo[2,1-b][1,3]thiazepine-7,4-diyl))bis(azanediyl))bis(1-oxopropane-1,2-diyl))bis(methylcarbamate) (80 mg, 0.065 mmol, 50.2% yield) as white solid. LCMS (2.5 min formic acid): Rt = 1.853 min, m/z: 511.8 {[(M-2Boc)+2]/2}⁺.
b. To a solution of tert-butyl ((S)-1-(((4S,7S,9aS)-8,8-dimethyl-5-oxo-7-(((1S,2R)-2-(prop-2-yn-1-yloxy)-2,3-dihydro-1H-inden-1-yl)carbamoyl)octahydropyrrolo[2,1-b][1,3]thiazepin-4-yl)amino)-1-oxopropan-2-yl)(methyl)carbamate (1.0 g, 1.63 mmol) and pyridine (775 mg, 9.79 mmol) in acetonitrile (20 mL) was added cupric acetate (366 mg, 1.96 mmol). The mixture was stirred at 85 °C for 1 h. The reaction mixture was cooled down, concentrated and diluted with ethyl acetate (100 mL). A solution of 20-fold ammonia (50 mL) was added. The water phase was extracted with ethyl acetate (2 × 50 mL). The organic layer was combined and washed with brine. Dried over anhydrous sodium sulfate and concentrated to get the crude. The residue was purified by chromatography on a silica gel column eluting with ethyl acetate / petroleum ether = 1/1 to give crude product. Then the crude product was purified by Prep-HPLC to get di-tert-butyl ((2S,2'S)-(((4S,4'S,7S,7'S,9aS,9a'S)-((((1S,1'S,2R,2'R)-(hexa-2,4-diyne-1,6-diylbis(oxy))bis(2,3-dihydro-1H-indene-2,1-diyl))bis(azanediyl))bis(carbonyl))bis(8,8-dimethyl-5-oxooctahydropyrrolo[2,1-b][1,3]thiazepine-7,4-diyl))bis(azanediyl))bis(1-oxopropane-1,2-diyl))bis(methylcarbamate) (800 mg, 0.65 mmol, 80.2% yield) as a white solid. *LCMS (ES, m*/*z): 1222.6, 512.2 [M*/*2-Boc+H]⁺, retention time 1.735 min. ¹H NMR (400 MHz, DMSO-d₆) δ ppm 7.96 (d, J = 8.8 Hz, 2H), 7.77 (d, J = 6.5 Hz, 2H), 7.24 (ddd, J = 14.9, 8.9, 2.8 Hz, 8H), 5.48 (t, J = 7.9 Hz, 2H), 5.38 (dd, J = 8.7, 5.3 Hz, 2H), 4.67 - 4.62 (m, 2H), 4.30 (m, 6H), 4.23 (s, 2H), 3.15 (t, J = 11.9 Hz, 2H), 3.03 (d, J = 4.4 Hz, 2H), 2.88 (d, J = 16.4 Hz, 2H), 2.73 (s, 6H), 2.26 - 2.17 (m, 2H), 2.16 - 2.05 (m, 6H), 1.76 (dt, J = 23.3, 10.1 Hz, 4H), 1.40 (s, 18H), 1.24 (d, J = 7.1 Hz, 6H), 1.07 (d, J = 14.9 Hz, 12H).*

### Step 4: (4S,4'S,7S,7'S,9aS,9a'S)-N,N'-((1S,1'S,2R,2'R)-(hexa-2,4-diyne-1,6-diylbis(oxy))bis(2,3-dihydro-1H-indene-2,1-diyl))bis(8,8-dimethyl-4-((S)-2-(methylamino)propanamido)-5-oxooctahydropyrrolo[2,1-b][1,3]thiazepine-7-carboxamide) dihydrochloride

To a solution of di-tert-butyl ((2S,2'S)-(((4S,4'S,7S,7'S,9aS,9a'S)-((((1S,1'S,2R,2'R)-(hexa-2,4-diyne-1,6-diylbis(oxy))bis(2,3-dihydro-1H-indene-2,1-diyl))bis(azanediyl))bis(carbonyl))bis(8,8-dimethyl-5-oxooctahydropyrrolo[2,1-b][1,3]thiazepine-7,4-diyl))bis(azanediyl))bis(1-oxopropane-1,2-diyl))bis(methylcarbamate) (80 mg, 0.065 mmol) in dichloromethane (5 mL) was added 4 N hydrogen chloride in 1,4-dioxane (1.5 mL). The mixture was stirred at room temperature overnight. The solvent was removed under reduced pressure and the solid was dried under high vacuum to afford (4S,4'S,7S,7'S,9aS,9a'S)-N,N'-((1S,1'S,2R,2'R)-(hexa-2,4-diyne-1,6-diylbis(oxy))bis(2,3-dihydro-1H-indene-2,1-diyl))bis(8,8-dimethyl-4-((S)-2-(methylamino)propanamido)-5-oxooctahydropyrrolo[2,1-b][1,3]thiazepine-7-carboxamide) dihydrochloride (55 mg, 0.050 mmol, 76.9 % yield) as white solid. ¹H NMR (400 MHz, DMSO-*d*₆) δ ppm 9.53-9.50 (m, 2H), 8.95-8.91 (m, 2H), 8.88 (d, J= 7.2 Hz, 2H), 8.00 (d, J= 8.8 Hz, 2H), 7.28-7.20 (m, 8H), 5.51 (t, J= 7.8 Hz, 2H), 5.38 (dd, J= 8.6, 5.4 Hz, 2H), 4.76-4.71 (m, 2H), 4.24-4.28 (m, 6H), 4.23 (s, 2H), 3.93-3.85 (m, 2H), 3.22-3.16 (m, 2H), 3.10-3.00 (m, 4H), 2.94-2.90 (m, 2H), 2.47 (t, J= 5.2 Hz, 6H), 2.29-2.14 (m, 4H), 1.87-1.79 (m, 4H), 1.42 (d, J= 6.8 Hz, 6H), 1.10 (s, 6H), 1.06 (s , 6H). LCMS (2.5 min formic acid): Rt = 1.328 min, m/z: 512.0 [(M+2)/2]⁺.

### Reference Example 1

### (4S,4'S,7S,7'S,9aS,9a'S)-N,N'-((1S,1'S,2R,2'R)-(hexane-1,6-diylbis(oxy))bis(2,3-dihydro-1H-indene-2,1-diyl))bis(8,8-dimethyl-4-((S)-2-(methylamino)propanamido)-5-oxooctahydropyrrolo[2,1-b][1,3]thiazepine-7-carboxamide) dihydrochloride

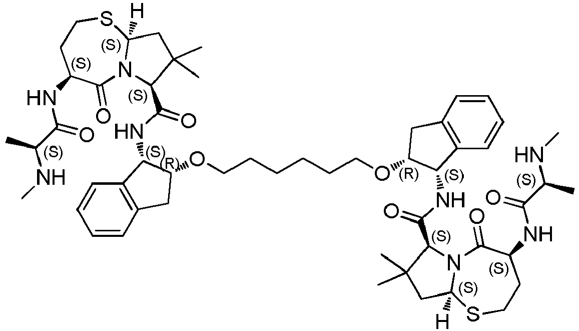

### Step 1: (1S,1'S,2R,2'R)-2,2'-(hexane-1,6-diylbis(oxy))bis(2,3-dihydro-1H-inden-1-amine)

To a solution of (1*S*,2*R*)-1-amino-2,3-dihydro-1H-inden-2-ol (1.35 g, 9.10 mmol) in tetrahydrofuran (80 mL) at 0 °C was added portionwise sodium hydride (60%, dispersion in Paraffin Liquid) (428 mg, 10.70 mmol). The mixture was allowed to warm to room temperature. 1,6-dibromohexane (1 g, 4.12 mmol) was then added. The resulting mixture was stirred at 70 °C overnight. The mixture was quenched with ice water (50 mL) and extracted with ethyl acetate (3 × 30 mL). The combined organic layers were washed with brine (50 mL), dried over anhydrous sodium sulfate, filtered and concentrated. The crude was purified by silica gel chromatography [ethyl acetate/methanol (10:1 v/v)] to afford (170 mg, 0.45 mmol, 10.9 % yield) as brown solid. LCMS (2.5 min formic acid): Rt = 1.284 min, m/z: 381.0 (M+1)⁺.

### Step 2: di-tert-butyl ((2S,2'S)-(((4S,4'S,7S,7'S,9aS,9a'S)-((((1S,1'S,2R,2'R)-(hexane-1,6-diylbis(oxy))bis(2,3-dihydro-1H-indene-2,1-diyl))bis(azanediyl))bis(carbonyl))bis(8,8-dimethyl-5-oxooctahydropyrrolo[2,1-b][1,3]thiazepine-7,4-diyl))bis(azanediyl))bis(1-oxopropane-1,2-diyl))bis(methylcarbamate)

To the mixture of (1*S*,1'*S*,2*R*,2'*R*)-2,2'-(hexane-1,6-diylbis(oxy)bis(2,3-dihydro-1H-inden-1-amine) (120 mg, 0.315 mmol) and (4S,7S,9aS)-4-((S)-2-((tert-butoxycarbonyl)(methyl)amino)propanamido)-8,8-dimethyl-5-oxooctahydropyrrolo[2,1-b][1,3]thiazepine-7-carboxylic acid (Intermediate I) (307 mg, 0.694 mmol) in 1,2-dichloroethane (8 mL) was added N-ethoxycarbonyl-2-ethoxy-1,2-dihydroquinoline (233 mg, 0.945 mmol) and *N*,*N*-diisopropylethylamine (162 mg, 1.26 mmol). The resulting mixture was stirred at 50 °C overnight. The solvent was removed under reduced pressure. The crude was purified by thin layer chromatography [ethyl acetate/petroleum ether (3/2 v/v)] followed by Prep-HPLC to give di-tert-butyl ((2S,2'S)-(((4S,4'S,7S,7'S,9aS,9a'S)-((((1S,1'S,2R,2'R)-(hexane-1,6-diylbis(oxy))bis(2,3-dihydro-1H-indene-2,1-diyl))bis(azanediyl))bis(carbonyl))bis(8,8-dimethyl-5-oxooctahydropyrrolo[2,1-b][1,3]thiazepine-7,4-diyl))bis(azanediyl))bis(1-oxopropane-1,2-diyl))bis(methylcarbamate) (60 mg, 0.049 mmol, 15.6% yield) as colorless oil. LCMS (2.5 min formic acid): Rt = 2.218 min, m/z: 515.9 {[(M-2Boc)+2]/2}⁺.

### Step 3: (4S,4'S,7S,7'S,9aS,9a'S)-N,N'-((1S,1'S,2R,2'R)-(hexane-1,6-diylbis(oxy))bis(2,3-dihydro-1H-indene-2,1-diyl))bis(8,8-dimethyl-4-((S)-2-(methylamino)propanamido)-5-oxooctahydropyrrolof2,1-b1f1,31thiazepine-7-carboxamide) dihydrochloride

To a solution of di-tert-butyl ((2S,2'S)-(((4S,4'S,7S,7'S,9aS,9a'S)-((((1S,1'S,2R,2'R)-(hexane-1,6-diylbis(oxy))bis(2,3-dihydro-1H-indene-2,1-diyl))bis(azanediyl))bis(carbonyl))bis(8,8-dimethyl-5-oxooctahydropyrrolo[2,1-b][1,3]thiazepine-7,4-diyl))bis(azanediyl))bis(1-oxopropane-1,2-diyl))bis(methylcarbamate) (60 mg, 0.049 mmol) in methanol (5 mL) was added 4 N hydrogen chloride in 1,4-dioxane (4 mL). The mixture was stirred at room temperature for 2 h. The solvent was removed under reduced pressure and the solid dried under high vacuum to give (4S,4'S,7S,7'S,9aS,9a'S)-N,N'-((1S,1'S,2R,2'R)-(hexane-1,6-diylbis(oxy))bis(2,3-dihydro-1H-indene-2,1-diyl))bis(8,8-dimethyl-4-((S)-2-(methylamino)propanamido)-5-oxooctahydropyrrolo[2,1-b][1,3]thiazepine-7-carboxamide) dihydrochloride (30 mg, 0.027 mmol, 55.1 % yield) as white solid. ¹H NMR (400 MHz, DMSO-*d*₆) δ ppm 9.33 (s, 2H), 8.87-8.85 (m, 4H), 7.91 (d, J= 8.4 Hz, 2H), 7.24-7.21 (m, 8H), 5.50 (t, J= 7.8 Hz, 2H), 5.35-5.31 (m, 2H), 4.76-4.72 (m, 2H), 4.24 (s, 2H), 4.11-4.10 (m, 2H), 3.91-3.87 (m, 2H), 3.72-3.65 (m, 2H), 3.51-3.47 (m, 2H), 3.39-3.37 (m, 4H), 3.19 (t, J= 12.4 Hz, 2H), 2.98-2.97 (m, 4H), 2.93-2.89 (m, 2H), 2.48 (s, 6H), 2.24-2.13 (m, 4H), 1.84-1.79 (m, 4H), 1.41-1.39 (m, 10H), 1.08 (m, 6H), 1.05 (m, 6H). LCMS (2.5 min formic acid): Rt = 1.365 min, m/z: 1030.8 (M+1)⁺.

### Reference Example 2

### (4S,4'S,7S,7'S,9aS,9a'S)-N,N'-((1S,1'S)-(hexa-2,4-diyne-1,6-diylbis(oxy))bis(1-phenylethane-2,1-diyl))bis(8,8-dimethyl-4-((S)-2-(methylamino)propanamido)-5-oxooctahydropyrrolo[2,1-b][1,3]thiazepine-7-carboxamide) dihydrochloride

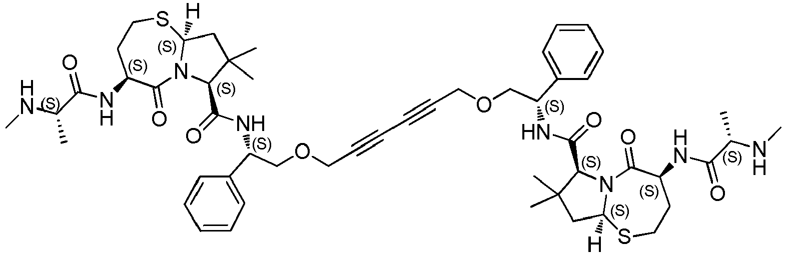

### Step 1: (S)-1-phenyl-2-(prop-2-yn-1-yloxy)ethan-1-amine

To a solution of (S)-2-amino-2-phenylethan-1-ol (6 g, 43.7 mmol) in dry tetrahydrofuran (200 mL) at 0 °C was added sodium hydride (60 %, dispersion in Paraffin Liquid) (1.9 g, 48.1 mmol). The mixture was stirred at 0 °C for 20 min. 3-Bromoprop-1-yne (5.72 g, 48.1 mmol) was then added. The mixture was heated at 70 °C overnight. Upon cooling, ice water (200 mL) was added to the mixture and the mixture was extracted with ethyl acetate (3 × 100 mL). The combined organic layers were washed with brine, dried over anhydrous sodium sulfate, filtered and concentrated. The crude was purified by silica gel chromatography [petroleum ether/ethyl acetate (1:1 v/v)] to afford (*S*)-1-phenyl-2-(prop-2-yn-1-yloxy)ethan-1-amine (2.5 g, 14.3 mmol, 32.65 % yield) as orange oil. ¹H NMR 1 (400 MHz, DMSO-*d*₆) δ ppm 7.44 - 7.14 (m, 5H), 4.18-4.12 (m, 2H), 4.08-4.00 (m, 1H), 3.55-3.47 (m, 1H), 3.44 - 3.37 (m, 2H), 1.84 (s, 2H).

### Step 2: tert-butyl ((S)-1-(((4S,7S,9aS)-8,8-dimethyl-5-oxo-7-(((S)-1-phenyl-2-(prop-2-yn-1-yloxy)ethyl)carbamoyl)octahydropyrrolo[2,1-b][1,3]thiazepin-4-yl)amino)-1-oxopropan-2-yl)(methyl)carbamate

To a solution of (S)-1-phenyl-2-(prop-2-yn-1-yloxy)ethan-1-amine (308 mg, 1.76 mmol) in dichloromethane (10 mL) was added (4S,7S,9aS)-4-((S)-2-((tert-butoxycarbonyl)(methyl)amino)propanamido)-8,8-dimethyl-5-oxooctahydropyrrolo[2,1-b][1,3]thiazepine-7-carboxylic acid (650 mg, 1.47 mmol), 2-(7-azabenzotriazol-1-yl)-N,N,N',N'-tetramethyluronium hexafluorophosphate (948 mg, 2.49 mmol) and N,N-diisopropyl-ethylamine (454 mg, 3.52 mmol) at room temperature. The mixture was stirred overnight. The reaction was quenched with water (300 mL), and extracted with dichloromethane (3 × 20 mL). The combined organic layers were washed with brine, dried, filtered and concentrated. The residue was purified by silica gel chromatography [ethyl acetate/petroleum ether (1/5 v/v)] to give tert-butyl ((S)-1-(((4S,7S,9aS)-8,8-dimethyl-5-oxo-7-(((S)-1-phenyl-2-(prop-2-yn-1-yloxy)ethyl)carbamoyl)octahydropyrrolo[2,1-b][1,3]thiazepin-4-yl)amino)-1-oxopropan-2-yl)(methyl)carbamate (194 mg, 0.32 mmol, 22.63 % yield) as yellow oil. LCMS (3.0 min formic acid): Rt = 1.70 min, m/z: 601 (M+1)⁺.

### Step 3: di-tert-butyl ((2S,2'S)-(((4S,4'S,7S,7'S,9aS,9a'S)-((3S,14S)-3,14-diphenyl-5,12-dioxa-2,15-diazahexadeca-7,9-diynedioyl)bis(8,8-dimethyl-5-oxooctahydropyrrolo[2,1-b][1,3]thiazepine-7,4-diyl))bis(azanediyl))bis(1-oxopropane-1,2-diyl))bis(methylcarbamate)

A mixture of tert-butyl ((S)-1-(((4S,7S,9aS)-8,8-dimethyl-5-oxo-7-(((S)-1-phenyl-2-(prop-2-yn-1-yloxy)ethyl)carbamoyl)octahydropyrrolo[2,1-b][1,3]thiazepin-4-yl)amino)-1-oxopropan-2-yl)(methyl)carbamate (194 mg, 0.32 mmol), copper(II) acetate (70.4 mg, 0.39 mmol) and pyridine (50.6 mg, 0.64 mmol) in acetonitrile (10 mL) was stirred at 85 °C for 1 h. Aqueous ammonia (20 fold dilution) was added to the mixture until it turned blue and the mixture was extracted with dichloromethane (3 × 30 mL). The combined organic layers were washed with brine, dried, filtered and concentrated. The residue was purified by thin layer chromatography [ethyl acetate/petroleum ether (2/1 v/v)] to give crude product (130 mg). This material was further purified by Prep-HPLC to afford di-tert-butyl ((2S,2'S)-(((4S,4'S,7S,7'S,9aS,9a'S)-((3S,14S)-3,14-diphenyl-5,12-dioxa-2,15-diazahexadeca-7,9-diynedioyl)bis(8,8-dimethyl-5-oxooctahydropyrrolo[2,1-b][1,3]thiazepine-7,4-diyl))bis(azanediyl))bis(1-oxopropane-1,2-diyl))bis(methylcarbamate) (70 mg, 0.058 mmol) as a white powder. LCMS (3.0 min formic acid): Rt = 2.10 min, m/z: 1199.7 (M+1)⁺.

### Step 4: (4S,4'S,7S,7'S,9aS,9a'S)-N,N'-((1S,1'S)-(hexa-2,4-diyne-1,6-diylbis(oxy))bis(1-phenylethane-2,1-diyl))bis(8,8-dimethyl-4-((S)-2-(methylamino)propanamido)-5-oxooctahydropyrrolo[2,1-b][1,3]thiazepine-7-carboxamide) dihydrochloride

To a solution of di-tert-butyl ((2S,2'S)-(((4S,4'S,7S,7'S,9aS,9a'S)-((3S,14S)-3,14-diphenyl-5,12-dioxa-2,15-diazahexadeca-7,9-diynedioyl)bis(8,8-dimethyl-5-oxooctahydropyrrolo[2,1-b][1,3]thiazepine-7,4-diyl))bis(azanediyl))bis(1-oxopropane-1,2-diyl))bis(methylcarbamate) (70 mg, 0.058 mmol) in dichloromethane (15 mL) was added hydrogen chloride (4 N in 1,4-dioxane, 1 mL). The reaction was stirred at room temperature overnight. The solvent was concentrated under reduced pressure and dried under high vacuum to give (4S,4'S,7S,7'S,9aS,9a'S)-N,N'-((1S,1'S)-(hexa-2,4-diyne-1,6-diylbis(oxy))bis(1-phenylethane-2,1-diyl))bis(8,8-dimethyl-4-((S)-2-(methylamino)propanamido)-5-oxooctahydropyrrolo[2,1-b][1,3]thiazepine-7-carboxamide) dihydrochloride (50 mg, 0.047 mmol, 79.36% yield) as white solid. 1H NMR (400 MHz, DMSO-*d*₆) δ ppm 9.24 (s, 2H), 8.86 (d, J = 6.4 Hz, 2H), 8.81 (d, J = 7.2 Hz, 2H), 8.38 (d, J = 8.0 Hz, 2H), 7.39-7.32 (m, 8H), 7.29-7.25 (m, 2H), 5.47 (t, J = 8.0 Hz, 2H), 5.04 (q, J = 6.8 Hz, 2H), 4.73-4.69 (m, 2H), 4.30 (s, 4H), 4.17 (s, 2H), 3.86 (q, J = 6.9 Hz, 2H), 3.62 (d, J= 6.0 Hz, 4H), 3.19-3.13 (m, 2H), 2.92-2.87 (m, 2H), 2.47 (s, 6H ), 2.22-2.17 (m, 2H), 2.13-2.09 (m, 2H), 1.87 (dd, J= 12.4, 8.8 Hz, 2H), 1.80-1.72 (m, 2H), 1.38 (d, J= 6.8 Hz, 6H), 1.06 (s, 6H), 1.01 (s , 6H). LCMS (2.5 min formic acid): Rt = 1.28 min, m/z: 999.7 (M+1)⁺.

### Reference Example 3

### (4S,4'S,7S,7'S,9aS,9a'S)-N,N'-((1S,1'S)-(hexane-1,6-diylbis(oxy))bis(1-phenylethane-2,1-diyl))bis(8,8-dimethyl-4-((S)-2-(methylamino)propanamido)-5-oxooctahydropyrrolo[2,1-b][1,3]thiazepine-7-carboxamide) dihydrochloride

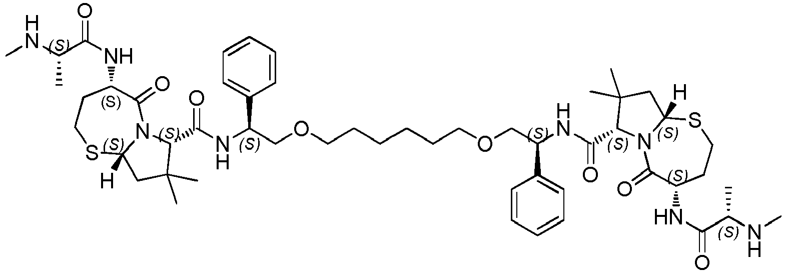

### Step 1: (1S,1'S)-2,2'-(hexane-1,6-diylbis(oxy))bis(1-phenylethan-1-amine)

To a mixture of (S)-2-amino-2-phenylethan-1-ol (1.0 g, 7.20 mmol) in dry tetrahydrofuran (20 mL) at 0 °C was added slowly portionwise sodium hydride (60 %, dispersion in Paraffin Liquid) (330 mg, 13.8 mmol) under nitrogen. The mixture was stirred at room temperature over 30 min and a solution of 1,6-dibromohexane (810 mg, 3.31 mmol) in dry tetrahydrofuran (10 mL) was added. The mixture was heated to reflux and stirred for another 5 h. Upon cooling, water (300 mL) was carefuly added to the mixture followed by extracted with dichloromethane (3 × 100 mL). The combined organic layers were washed with brine, dried, filtered and concentrated. The residue was purified by thin layer chromatography (dichloromethane/methanol (7:1 v/v)] to give (1S,1'S)-2,2'-(hexane-1,6-diylbis(oxy))bis(1-phenylethan-1-amine) (410 mg, 1.15 mmol, 25.6 % yield) as yellow oil. LCMS (2.5 min formic acid): Rt = 1.24 min, m/z: 357.0 (M+1)⁺.

### Step 2: di-tert-butyl ((2S,2'S)-(((4S,4'S,7S,7'S,9aS,9a'S)-((3S,14S)-3,14-diphenyl-5,12-dioxa-2,15-diazahexadecanedioyl)bis(8,8-dimethyl-5-oxooctahydropyrrolo[2,1-b][1,3]thiazepine-7,4-diyl))bis(azanediyl))bis(1-oxopropane-1,2-diyl))bis(methylcarbamate)

A solution of (1S,1'S)-2,2'-(hexane-1,6-diylbis(oxy))bis(1-phenylethan-1-amine) (90 mg, 0.25 mmol), (4S,7S,9aS)-4-((S)-2-((tert-butoxycarbonyl)(methyl)amino)propanamido)-8,8-dimethyl-5-oxooctahydropyrrolo[2,1-b][1,3]thiazepine-7-carboxylic acid (235.2 mg, 0.53 mmol), N-ethoxycarbonyl-2-ethoxy-1,2-dihydroquinoline (187.3 mg, 0.76 mmol) and N,N-diisopropyl-ethylamine (130.5 mg, 1.01 mmol) in 1,2-dichloroethane (5 mL) was stirred at 50 °C overnight. The solvent was removed under reduced pressure. The residue was purified by thin layer chromatography [ethyl acetate/petroleum ether (1:1 v/v)] to give crude product (120 mg) as yellow oil. This material was further purified by Prep-HPLC to give di-tert-butyl ((2S,2'S)-(((4S,4'S,7S,7'S,9aS,9a'S)-((3S,14S)-3,14-diphenyl-5,12-dioxa-2,15-diazahexadecanedioyl)bis(8,8-dimethyl-5-oxooctahydropyrrolo[2,1-b][1,3]thiazepine-7,4-diyl))bis(azanediyl))bis(1-oxopropane-1,2-diyl))bis(methylcarbamate) (50 mg, 0.041 mmol, 16.4 % yield) as a white solid. LCMS (2.5 min formic acid): Rt = 1.89 min, m/z: 503.9 {[(M-2Boc)+2]/2}⁺.

### Step 3: (4S,4'S,7S,7'S,9aS,9a'S)-N,N'-((1S,1'S)-(hexane-1,6-diylbis(oxy))bis(1-phenylethane-2,1-diyl))bis(8,8-dimethyl-4-((S)-2-(methylamino)propanamido)-5-oxooctahydropyrrolof2,1-b1f1,31thiazepine-7-carboxamide) dihydrochloride

To a solution of di-tert-butyl ((2S,2'S)-(((4S,4'S,7S,7'S,9aS,9a'S)-((3S,14S)-3,14-diphenyl-5, 12-dioxa-2, 15-diazahexadecanedioyl)bis(8,8-dimethyl-5-oxooctahydropyrrolo[2, 1-b][1,3]thiazepine-7,4-diyl))bis(azanediyl))bis(1-oxopropane-1,2-diyl))bis(methylcarbamate) (100 mg, 0.083 mmol) in dichloromethane (5 mL) was added hydrogen chloride (4 N in 1,4-dioxane, 1 mL). The mixture was stirred at room temperature overnight. The mixture was concentrated under reduced pressure and dried under high vacuum to give (4S,4'S,7S,7'S,9aS,9a'S)-N,N'-((1S,1'S)-(hexane-1,6-diylbis(oxy))bis(1-phenylethane-2,1-diyl))bis(8,8-dimethyl-4-((S)-2-(methylamino)propanamido)-5-oxooctahydropyrrolo[2,1-b][1,3]thiazepine-7-carboxamide) dihydrochloride (41.9 mg, 0.038 mmol, 45.8 % yield) as a white solid. ¹H NMR (400 MHz, DMSO-*d*₆) δ ppm 9.34 (s, 2H), 8.88-8.86 (m, 2H), 8.82 (d, J= 7.8 Hz, 2H), 8.36 (d, J= 8.4 Hz, 2H), 7.38-7.31 (m, 8H), 7.27-7.23 (m, 2H), 5.47 (t, J= 7.8 Hz, 2H), 5.48-5.02 (m, 2H), 4.73-4.68 (m, 2H), 4.17 (s, 2H), 3.88-3.83 (m, 2H), 3.72-3.65 (m, 2H), 3.51-3.47 (m, 6H), 3.32-3.28 (m, 4H), 3.19-3.13 (m, 2H), 2.90-2.87 (m, 2H), 2.46 (t, J= 4.4H, 6H), 2.22-2.17 (m, 2H), 2.13-2.09 (m, 2H), 1.89-1.84 (m, 2H), 1.79-1.34 (m, 2H), 1.39-1.38 (m, 10H), 1.06 (s, 6H), 1.01 (m, 6H). LCMS (2.5 min formic acid): Rt = 1.258 min, m/z: 1006.8 (M+1)⁺.

### Reference Example 4

### (4S,4'S,7S,7'S,9aS,9a'S)-N,N'-((1S,1'S)-((1,4-phenylenebis(methylene))bis(oxy))bis(1-phenylethane-2,1-diyl))bis(8,8-dimethyl-4-((S)-2-(methylamino)propanamido)-5-oxooctahydropyrrolo[2,1-b][1,3]thiazepine-7-carboxamide) dihydrochloride

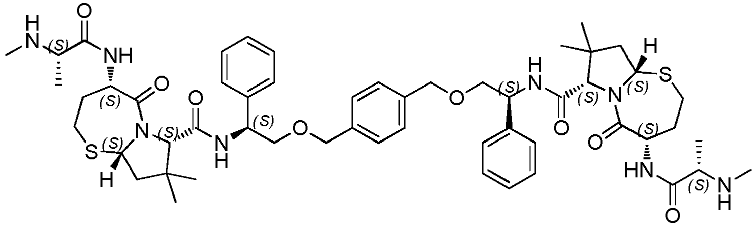

### Step 1: (1S,1'S)-2,2'-((1,4-phenylenebis(methylene))bis(oxy))bis(1-phenylethan-1-amine)

To a solution of (S)-2-amino-2-phenylethan-1-ol (2.2 g, 16.0 mmol) in dry tetrahydrofuran (30 mL) was added sodium hydride (60%, dispersion in Paraffin Liquid) (760 mg, 31.6 mmol). After 30 minutes, 1,4-bis-bromomethyl-benzene (2.0 g, 7.6 mmol) was added. The mixture was stirred at room temperature overnight. The mixture was quenched with ice water (50 mL) and extracted with ethyl acetate (3 × 60 mL). The combined organic layers were washed with brine, dried (Na₂SO₄), filtered and concentrated. The crude was purified by silica gel chromatography (dichloromethane/methanol (20:1 v/v)] to afford (1S,1'S)-2,2'-((1,4-phenylenebis(methylene))bis(oxy))bis(1-phenylethan-1-amine) (1.2 g, 3.2 mmol, 31% yield) as yellow oil. LCMS (2.5 min formic acid): Rt = 1.14 min, m/z: 377.0 (M+1)⁺.

### Step 2: tert-butyl di-tert-butyl ((2S,2'S)-(((4S,4'S,7S,7'S,9aS,9a'S)-((((1S,1'S)-((1,4-phenylenebis(methylene))bis(oxy))bis(1-phenylethane-2,1-diyl))bis(azanediyl))bis(carbonyl))bis(8,8-dimethyl-5-oxooctahydropyrrolo[2,1-b][1,3]thiazepine-7,4-diyl))bis(azanediyl))bis(1-oxopropane-1,2-diyl))bis(methylcarbamate)

A solution of (1S,1'S)-2,2'-((1,4-phenylenebis(methylene))bis(oxy))bis(1-phenylethan-1-amine) (100 mg, 0.266 mmol), (4S,7S,9aS)-4-((S)-2-((tert-butoxycarbonyl)(methyl)amino)propanamido)-8,8-dimethyl-5-oxooctahydropyrrolo[2,1-b][1,3]thiazepine-7-carboxylic acid (247.5 mg, 0.56 mmol), N-ethoxycarbonyl-2-ethoxy-1,2-dihydroquinoline (197.1 mg, 0.80 mmol) and *N*,*N*-diisopropyl-ethylamine (137.3 mg, 0.29 mmol) in 1,2-dichloroethane (6 mL) was stirred at 50 °C overnight. The mixture was concentrated under reduced pressure and purified by thin layer chromatography [ethyl acetate/petroleum ether (2:1 v/v)] to give crude product (200 mg) as yellow oil. This material was further purified by Prep-HPLC to give tert-butyl di-tert-butyl ((2S,2'S)-(((4S,4'S,7S,7'S,9aS,9a'S)-((((1S,1'S)-((1,4-phenylenebis(methylene))bis(oxy))bis(1-phenylethane-2,1-diyl))bis(azanediyl))bis(carbonyl))bis(8,8-dimethyl-5-oxooctahydropyrrolo[2,1-b][1,3]thiazepine-7,4-diyl))bis(azanediyl))bis(1-oxopropane-1,2-diyl))bis(methylcarbamate) (160 mg, 0.130 mmol, 49.1% yield) as white solid. LCMS (2.5 min formic acid): Rt = 1.86 min, m/z: 514.1 {[(M-2Boc)+2]/2}⁺.

### Step 3: (4S,4'S,7S,7'S,9aS,9a'S)-N,N'-((1S,1'S)-((1,4-phenylenebis(methylene))bis(oxy))bis(1-phenylethane-2,1-diyl))bis(8,8-dimethyl-4-((S)-2-(methylamino)propanamido)-5-oxooctahydropyrrolo[2,1-b][1,3]thiazepine-7-carboxamide) dihydrochloride

To a solution of di-tert-butyl ((2S,2'S)-(((4S,4'S,7S,7'S,9aS,9a'S)-((((1S,1'S)-((1,4-phenylenebis(methylene))bis(oxy))bis(1-phenylethane-2,1-diyl))bis(azanediyl))bis(carbonyl))bis(8,8-dimethyl-5-oxooctahydropyrrolo[2,1-b][1,3]thiazepine-7,4-diyl))bis(azanediyl))bis(1-oxopropane-1,2-diyl))bis(methylcarbamate) (160 mg, 0.13 mmol) in dichloromethane (15 mL) was added hydrogen chloride (4 N in 1,4-dioxane, 1 mL). The reaction was stirred at room temperature overnight. The mixture was concentrated under reduced pressure and dried under high vacuum to give (4S,4'S,7S,7'S,9aS,9a'S)-N,N'-((1S,1'S)-((1,4-phenylenebis(methylene))bis(oxy))bis(1-phenylethane-2,1-diyl))bis(8,8-dimethyl-4-((S)-2-(methylamino)propanamido)-5-oxooctahydropyrrolo[2,1-b][1,3]thiazepine-7-carboxamide) dihydrochloride (110 mg, 0.11 mmol, 78 % yield) as white solid. ¹H NMR (400 MHz, DMSO-*d*₆) δ ppm 9.39 (s, 2H), 8.88-8.86 (m, 2H), 8.81 (d, J= 6.8 Hz, 2H), 8.42 (d, J= 8.0 Hz, 2H), 7.39-7.32 (m,8H), 7.28-7.25 (m, 2H), 7.20 (s, 4H), 5.47 (t, J= 7.6 Hz, 2H), 5.08 (q, J= 6.0 Hz, 2H), 4.70 (t, J= 9.0 Hz, 2H), 4.49-4.43 (m, 4H), 4.18 (s, 2H), 3.86 (s, 2H), 3.72-3.63 (m, 2H), 3.55-3.47 (m, 2H), 3.13 (t, J= 12.2 Hz, 2H), 2.82-2.78 (m, 2H), 2.46 (s, 6H), 2.21-2.16 (m, 2H), 2.09-2.06 (m, 2H), 1.88-1.83 (m, 2H), 1.77-1.68 (m, 2H), 1.39 (d, J= 6.8 Hz, 6H), 1.05 (m, 6H), 0.97 (m, 6H). LCMS (2.5 min formic acid): Rt = 1.334 min, m/z: 1027.8 (M+1)⁺.

### • Jurkat latency reversal assay.

Jurkat HIV-luciferase clones were maintained in RPMI medium 1640 (Gibco by Life Technologies) containing 10% (vol/vol) fetal bovine serum (SAFC/Sigma-Aldrich) and 25 units/mL penicillin, 25 units/mL streptomycin (Gibco by Life Technologies), and were split 1:4 every 3 to 4 days to maintain a cell density of ~0.3 to 1 million cells/mL. The Jurkat clones were maintained with the addition of 500nM EFV in the medium. Three Jurkat cell clones (C16, I15, and N6), each harboring one or two integrated HIV proviruses expressing the luciferase reporter gene, were added at equal amounts for a total of 5,000 cells per well to 384-well plates containing compound titrations. Dose-response testing was performed on compounds dissolved in dimethyl sulfoxide (DMSO; Fisher Scientific, Merelbeke, Belgium) dispensed in duplicate serial 3-fold, 14-point titrations using a D300e Digital Droplet Dispenser (Hewlett-Packard) to give final assay concentrations of 10 µM to 2.1 pM in 50 µL of medium at 0.5% DMSO (vol/vol) final concentration. Cells and compound were incubated at 37°C for 48 hours, unless otherwise indicated, followed by the addition of 20 µL of Steady-Glo^{®} Luciferase (Promega). Luminescence resulting from the induction of the virally expressed luciferase was measured using an EnVision 2102 Multilabel Plate Reader (Perkin Elmer). Dose-response relationships were analyzed with GraphPad PRISM 6 using a four-parameter logistic regression model to calculate the concentration of compound that gives half-maximal response (EC₅₀) and the maximal percent activation compared to the vehicle control.

Results from the above assay are set forth in Table 3.

**Table 3**

| **Example No.** | **EC50 (nM)** |
|---|---|
| 1 | 9.3 |
| Reference Example 1 | 11.9 |
| Reference Example 2 | 5.5 |
| Reference Example 3 | 6.1 |
| Reference Example 4 | 6.8 |

### Pharmacokinetic Data

Rodent pharmacokinetic (PK) data of several SMAC mimetic compounds was compared with that of SMACm AZD5582 PK data (Figure 1). As can be seen in Figure 1, the plasma drug concentration versus time curves for the compounds demonstrate a PK relationship comparable to that of AZD5582.

### NFkB2 Gene Induction

5X10⁵ normal donor CD4 T cells were treated for 24h with serial dilutions of compounds starting at 1.0µM at 6 fold, 7 places. Total RNA was isolated using the RNEasy Mini kit (Qiagen) per the manufacturer's instructions. The following TaqMan primer probe sets were sourced from Applied Biosystems: Hs00174517_m1 (NFKB2) and Hs02800695 (HPRT1). TaqMan-based real-time PCR (Fast Virus 1-Step Master Mix, Applied Biosystems) was used to amplify host genes of interest. Gene expression was normalized to HPRT1 and comparative threshold cycle (CT) method (ΔΔCT) was used for relative fold change of gene expression as compared to untreated cells. The data was analyzed by QuantStudio3 Real-Time PCR System. Results are provided in Table 4.

### p100/p52 WB line

### Cell Culture and Treatment

Cell cultures were set up to evaluate several SMACm compounds. Frozen PBMC cells from healthy donors were thawed and plated in 2 ml deepwell plates at 2x10⁶ cells/ml. Serial dilutions of each compound was carried out as a 7 fold 1:6 dilution with the starting final dose of 1000nM. Cells were then combined with compound and place at 37° for 24hrs. The following day cells were pelleted and placed at -80° until lysis.

### Harvest of Whole Cell Lysis

In preparation for cell lysis, the frozen cell pellets and an aliquot of NP40 Cell Lysis Buffer (Invitrogen, Part# FNN0021), as well as an aliquot of 10x Protease Inhibitor Cocktail (Sigma, Part# P-2714) are thawed on ice. Once thawed, the complete lysis buffer is prepared by adding 10% final volume PI cocktail to the NP40 buffer and then adding in sufficient volume of 0.1 M PMSF (Sigma, Part# 93482) and 1 M DTT (Sigma, Part# 43816) to create a final concentration of 1 mM each in the complete lysis buffer. Cell lysis is then carried out by resuspending each cell pellet in 30 µL per 10^6 cells then incubating on ice for 30 minutes with vigorous vortexing every 10 minutes. The lysed cells are then centrifuged at 13K RPM for 10 minutes at 4°C in a refrigerated microcentrifuge. The supernatants (lysates) are then transferred into new microcentrifuge tubes and either stored at -80° or utilized immediately for protein concentration and Western blot analysis.

### Bradford Microplate Procedure

Protein concentration for each lysate is determined using a detergent compatible Bradford assay (Pierce, Part# 23246) following the manufacturer's microplate instructions.

### Protein Separation and Immunodetection

Capillary Western analysis were performed using the ProteinSimple Jess System. A total of 1ug of cell lysate was prepared according to the provided protocol supplied by ProteinSimple. The primary antibody for p100/p52 (Cell, Signaling #3017) is diluted 1:10 for use in the Jess system. The 12-230 kDa Jess Seperation Module capillary cartridges Separation Module (SM-W004) is used in conjunction with the Anti-Rabbit Secondary NIR conjugate ( 043-819). The Separation module includes prefilled plates that the samples, primary and secondary antibodies, as well as protein normalization reagent and diluents are loaded onto to perform separation and detection. Once the assay has run, the data is then analyzed utilizing the Compass software provided by ProteinSimple.

### Immunoblot Analysis

Alternatively to capillary electrophoresis, traditional immunoblot assays were carried out, 10 µg of cell lysate was loaded per well into 4-20% Tris-Glycine SDS-PAGE gels. Protein from the SDS-PAGE gels were transferred to Turbo Midi PVDF Transfer Packs (BioRad) using the "Mixed MW" protocol for one Midi Format Gel (constant 2.5A up to 25V, for 7 minutes) of the Trans-Blot Turbo Transfer System (Bio-Rad) with premade Trans-Blot per the manufacturer's instructions. After transfer, PVDF membranes were blocked in 5% bovine serum albumin (BSA) in 1x TBS (BioRad) with 0.1% Tween-20 for 1 hour at room temperature with gentle rocking. Primary antibodies were added and incubated overnight at 4°C (anti-p100/p52, Cell Signaling Technology #3017, 1:1000; anti-Actin-HRP conjugate, Abcam #49900, 1:30,000). Following primary staining, the membrane washed three times with 1x Tris Buffered Saline (TBS)+0.1% TWEEN^{®} 20 for 10 minutes each wash. After washing, the membrane was incubated in 5% BSA in 1x TBS+0.1% TWEEN^{®} 20 with the appropriate secondary antibody for 2 hours at room temperature. Following secondary stain the membrane was washed twice for 10 minutes with 1x TBS+0.1% TWEEN^{®} 20 followed by a 10-minute wash with 1x TBS. The membrane was then patted dry with filter paper and an image was captured of the undeveloped membrane on the ChemiDoc MP Imaging System using Image Lab software (BioRad). Sufficient ECL reagent (GE Healthcare) was used to cover the membrane and a series of images were taken starting with 0.001 second and doubling to tripling the exposure time until the luminescence from the developed membrane saturated the image. The developed membrane was then washed three times with 1x TBS for 5 minutes to remove the residual ECL reagent and then stored at 4°C in sufficient 1x TBS to submerse the entire membrane. Densitometry of images of the developed membrane were then carried out using the Image Lab software. Some membranes were stripped for one minute with One Minute Plus Western Blot Stripping Buffer (GM Biosciences) and then washed three times for 10 minutes with 1x TBS. The stripped membranes were then blocked in 5% BSA in 1x TBS+0.1% TWEEN^{®} 20 for an hour and reprobed overnight with a new primary antibody. Results of the p100/p52 WB line assay are provided in Table 4.

### Cell Associated HIV RNA (caRNA)

During the productive phase of the viral life cycle, HIV produces a large number of differentially spliced transcripts collectively termed cell-associated HIV RNA (ca-HIV RNA) within some cells that are infected. In HIV-infected individuals on suppressive antiretroviral therapy (ART), changes in the levels of caHIV RNA is an accepted surrogate measure of the efficacy of HIV latency reversal.

To measure ca-HIVRNA, peripheral blood mononuclear cells (PBMCs) are isolated from leukocytes obtained by continuous-flow leukapheresis. Total T cells were isolated from PBMCs by negative selection using the EasySep Human T cell Enrichment kit (StemCell, Vancouver, Canada) per the manufacturer's recommendations. Resting T cells are isolated by negative selection with an immunomagnetic column as described previously (25) and either cryopreserved immediately or maintained for two days in IMDM medium (Gibco), 10% FBS, 2µg/mL IL-2 (Peprotech), and antiretrovirals to prevent viral expansion.

For SMACm treatment, three to five replicates of 2-5×10⁶ CD4⁺T cells were treated for 48 hours at 37°C in 1mL of RPMI medium 1640, 10% FBS, 10µg/mL of enfuvirtide (Sigma), and 200 nM rilpivirine. 10nM phorbol 12-myristate 13-acetate (PMA; Sigma) with 1µM ionomycin (Sigma) was used as a positive control for LRA activation and 0.2% DMSO vehicle was used as a negative control. Following treatment, cells were lysed and RNA and DNA were co-extracted using an AIIPrep 96 RNA/DNA kit (Qiagen, Valencia, CA) per the manufacturer's instructions, adjusting the volume of lysis buffer to 0.6mL, adding an on-column DNase I treatment (Qiagen) and eluting RNA in 50µL of water.

RT-qPCR was performed in triplicate for each of three replicate wells using TaqMan Fast Virus 1-step RT-qPCR Master Mix (Applied Biosciences) with 5µL isolated RNA and 900nM of HIV capsid primers HIV-gag (5'-ATCAAGCAGCTATGCAAATGTT-3' (SEQ ID NO: 1)) and gag reverse (5'-CTGAAGGGTACTAGTAGTTCCTGCTATGTC-3' (SEQ ID NO: 2)) and 250nM of FAM/ZEN/IABFQ HIV gag probe (5'-ACCATCAATGAGGAAGCTGCAGAATGGGA-3' (SEQ ID NO: 3)). Samples were amplified and data was collected using a QuantStudio^{™} 3 Real-Time PCR system (Applied Biosystems) with the following cycling conditions: one cycle at 50°C for 5 minutes (reverse transcription), one cycle at 95°C for 20 seconds (reverse transcriptase inactivation), and 50 cycles at 95°C for 3 seconds and 60°C for 20 seconds (denaturation and annealing/extension).

HIV absolute HIV gag RNA copies per reaction were determined using an HIV gag gBlock qPCR standard corresponding to the DNA sequence of the qPCR product (5'-ATCAAGCAGCCATGCAAATGTTAAAAGAGACCATCAATGAGGAAGCTGCAGAATGGGAT AGATTGCATCCAGTGCATGCAGGGCCTATTGCACCAGGCCAGATGAGAGAACCAAGGG GAAGTGACATAGCAGGAACTACTAGTACCCTTCAG-3' (SEQ ID NO: 4); Integrated DNA Technologies), and copies were normalized to cell counts as determined by bright field microscopy. RT-qPCR efficiency was required to be between 90% to 110%. Assay values with a positive signal that were less than the lower limit of detection (LLOD) of seven HIV gag copies per reaction were adjusted to the LLOD. Analysis was performed using QuantStudio^{™} Design and Analysis Software (Applied Biosystems) and the R software package (described in detail below).

Shown in Table 4 are data comparing the Jurkat EC50, p100/p52 WB assay, NFkB2 gene induction assay and caRNA assay data of several SMAC mimetics disclosed herein with competitor mimetics.

Compounds of the present invention (namely Reference Examples 1 to 4 and Example 1), are potent dimeric SMACm which are able to activate the ncNF-kB pathway and induce HIV expression. These molecules efficiently deplete clAP1 and clAP2, lead to cleavage of p100 to release p52, activate the ncNF-kB pathway and induce HIV expression. Moreover, these have properties that engage the ncNF-kB pathway in vivo. In particular, Example 1 and Reference Example 1 are believed to possess the best combination of unexpected and surprising properties.

**Table 4: Summary of SMAC mimetics disclosed herein with other mimetics**

| | **AZD-5582** | **Birinapant** | **SBI-0637142** | **Formula F** US20190135861A1 | **Ref Ex. 2** | **Ex. 1** | **Ref Ex. 4** | **Ref Ex. 3** | **Ref Ex. 1** |
|---|---|---|---|---|---|---|---|---|---|
| Jurkat EC50 (nM) | 8 | 200 | 1 | ^{~}1000 | 5.5 | 9.3 | 6.8 | 6.1 | 11.9 |
| p100/p52 Western Blot (WB)EC50 (nM) | 63 | 1000 | 100-1000 | >1000 | 158 | 12.6 | 3.1 | 1 | 3.1 |
| NFkB2 gene induction EC50 (nM) | 28 | >1000 | 18000 | Not tested | 46 | 73 | 3.6 | 11 | 75 |
| caRNA Lowest efficacious concentration observed | 10 | >1000 | >1000 | Not tested | 10 | 10 | 10 | 10 | 10 |

| | | | | | | | | | |
|---|---|---|---|---|---|---|---|---|---|
| "Ex" means Example | | | | | | | | | |

wherein: 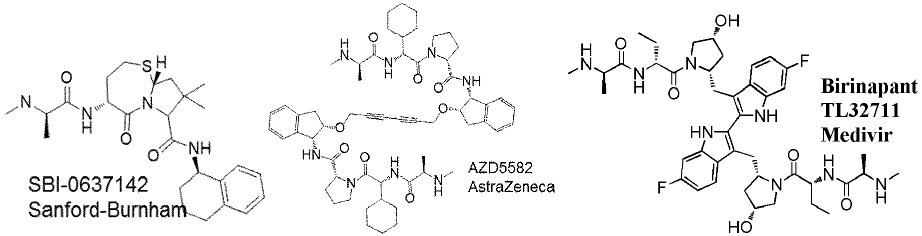 and SBI-described dimer Formula F:

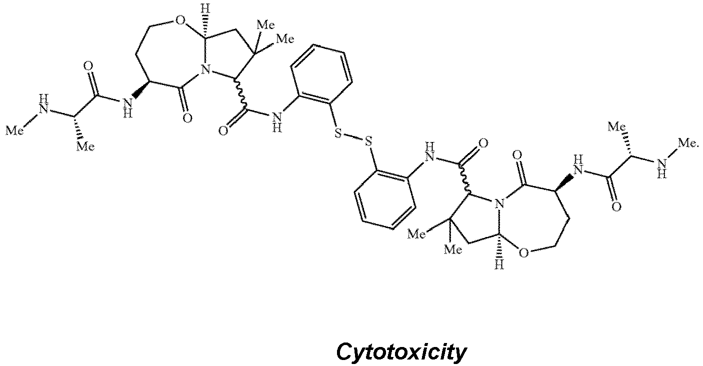

Cell viability of Jurkat cells was determined using the CellTiter-Glo^{®} Luminescent Cell Viability Assay (Promega), a homogeneous method to determine the ATP levels in a culture well, which corresponds to the presence of metabolically active cells in culture. Cells were cultured as indicated elsewhere for the Jurkat assays. For assessment of SMACm-mediated toxicity in the context of TNFa 10ng/mL TNFa (R&D Systems) was added to the culture A proportion of cells was removed and 30 µL of Promega CellTiter-Glo^{®} reagent was added to each well containing cells and luminescence was measured using a Perkin Elmer EnVision plate reader. Dose-response relationships were analyzed with GraphPad PRISM 6 using a four-parameter model logistic regression model to calculate the concentration of compound that reduces cell viability by 50% when compared to untreated controls (CC₅₀).

Efforts have been made to ensure accuracy with respect to numbers used (e.g. amounts, temperature, etc.) but some experimental errors and deviations should be accounted for.

One skilled in the art will recognize many methods and materials similar or equivalent to those described herein, which could be used in the practicing the subject matter described herein. The present disclosure is in no way limited to just the methods and materials described.

Unless defined otherwise, technical and scientific terms used herein have the same meaning as commonly understood by one of ordinary skill in the art to which this subject matter belongs, and are consistent with: Singleton et al (1994) Dictionary of Microbiology and Molecular Biology, 2nd Ed., J. Wiley & Sons, New York, NY; and Janeway, C., Travers, P., Walport, M., Shlomchik (2001) Immunobiology, 5th Ed., Garland Publishing, New York.

Throughout this specification and the claims, the words "comprise," "comprises," and "comprising" are used in a non-exclusive sense, except where the context requires otherwise. It is understood that embodiments described herein include "consisting of" and/or "consisting essentially of" embodiments.

As used herein, the term "about," when referring to a value is meant to encompass variations of, in some embodiments ± 50%, in some embodiments ± 20%, in some embodiments ± 10%, in some embodiments ± 5%, in some embodiments ± 1%, in some embodiments ± 0.5%, and in some embodiments ± 0.1% from the specified amount, as such variations are appropriate to perform the disclosed methods or employ the disclosed compositions.

Where a range of values is provided, it is understood that each intervening value, to the tenth of the unit of the lower limit, unless the context clearly dictates otherwise, between the upper and lower limit of the range and any other stated or intervening value in that stated range, is encompassed. The upper and lower limits of these small ranges which may independently be included in the smaller rangers is also encompassed, subject to any specifically excluded limit in the stated range. Where the stated range includes one or both of the limits, ranges excluding either or both of those included limits are also included.

## Claims

1. A compound having the structure: 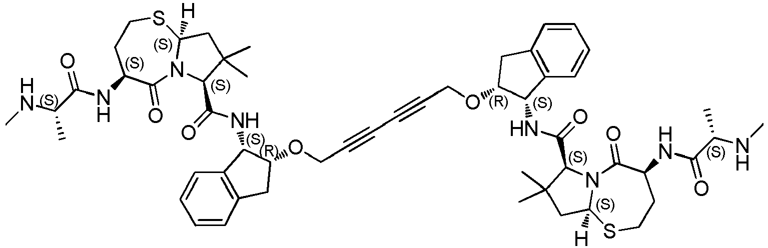 or a pharmaceutically acceptable salt thereof.

2. A salt according to Claim 1, wherein the salt is present as a hydrochloride salt.

3. The salt according to Claim 2, wherein the hydrochloride salt is a dihydrochloride salt.

4. A pharmaceutical composition comprising a compound according to Claim 1, or a pharmaceutically acceptable salt thereof, and a pharmaceutically acceptable excipient.

5. A compound of Claim 1, or a pharmaceutically acceptable salt thereof, for use in depleting latent HIV infected cells.

6. A compound of Claim 1, or a pharmaceutically acceptable salt thereof, for use in treating an HIV infection.

7. A compound of Claim 1 having the structure:

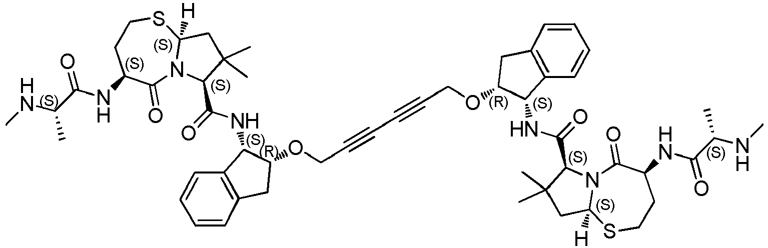

8. A pharmaceutical composition comprising a compound according to Claim 7, and a pharmaceutically acceptable excipient.

9. A compound of Claim 7 for use in depleting latent HIV infected cells.

10. A compound of Claim 7 for use in treating an HIV infection.

11. A compound of Claim 1 or a pharmaceutically acceptable salt thereof for use in medical therapy.

12. A compound of Claim 1 or a pharmaceutically acceptable salt thereof and one or more additional agents active against HIV for use in depleting HIV infected cells.

## Patentansprüche

1. Verbindung mit der Struktur: 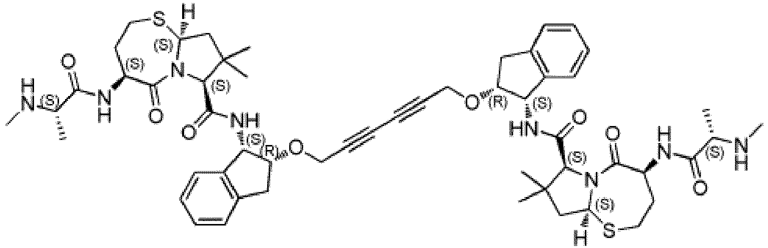 oder ein pharmazeutisch annehmbares Salz hiervon.

2. Salz nach Anspruch 1, wobei das Salz als ein Hydrochloridsalz vorliegt.

3. Salz nach Anspruch 2, wobei das Hydrochlorid-Salz ein Dihydrochloridsalz ist.

4. Pharmazeutische Zusammensetzung, umfassend eine Verbindung nach Anspruch 1, oder ein pharmazeutisch annehmbares Salz hiervon, und einen pharmazeutisch annehmbaren Hilfsstoff.

5. Verbindung nach Anspruch 1, oder ein pharmazeutisch annehmbares Salz hiervon, zur Anwendung bei der Depletion latenter HIV-infizierter Zellen.

6. Verbindung nach Anspruch 1, oder ein pharmazeutisch annehmbares Salz hiervon, zur Anwendung bei der Behandlung einer HIV-Infektion.

7. Verbindung nach Anspruch 1 mit der Struktur:

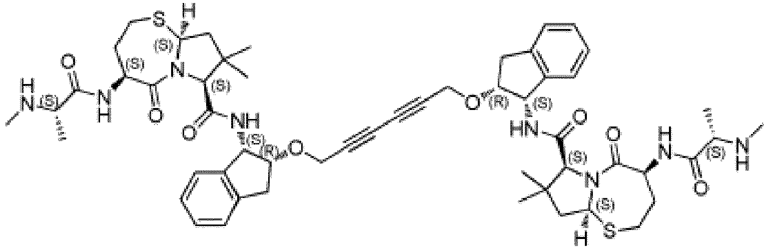

8. Pharmazeutische Zusammensetzung, umfassend eine Verbindung nach Anspruch 7 und ein pharmazeutisch annehmbares Hilfsmittel.

9. Verbindung nach Anspruch 7 zur Anwendung bei der Depletion latenter HIV-infizierter Zellen.

10. Verbindung nach Anspruch 7 zur Anwendung bei der Behandlung einer HIV-Infektion.

11. Verwendung nach Anspruch 1 oder ein pharmazeutisch annehmbares Salz hiervon zur Anwendung bei der medizinischen Therapie.

12. Verbindung nach Anspruch 1 oder ein pharmazeutisch annehmbares Salz hiervon und ein oder mehrere zusätzliche Wirkstoffe gegen HIV zur Anwendung bei der Depletion HIVinfizierte Zellen.

## Revendications

1. Composé ayant la structure : 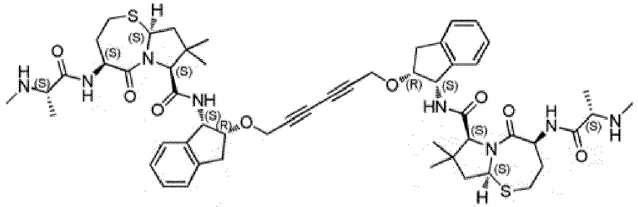 ou un de ses sels pharmaceutiquement acceptables

2. Sel selon la revendication 1, dans lequel le sel est présent sous forme de sel de chlorhydrate.

3. Sel selon la revendication 2, dans lequel le sel de chlorhydrate est un sel de dichlorhydrate.

4. Composition pharmaceutique comprenant un composé selon la revendication 1, ou un de ses sels pharmaceutiquement acceptables, et un excipient pharmaceutiquement acceptable.

5. Composé selon la revendication 1, ou un de ses sels pharmaceutiquement acceptables, pour l'utilisation dans l'élimination des cellules infectées par le VIH latent.

6. Composé selon la revendication 1, ou un de ses sels pharmaceutiquement acceptables, pour l'utilisation dans le traitement d'une infection par le VIH.

7. Composé selon la revendication 1 ayant la structure :

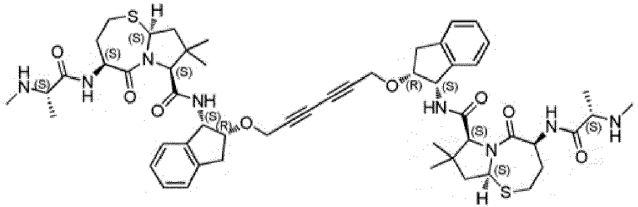

8. Composition pharmaceutique comprenant un composé selon la revendication 7, et un excipient pharmaceutiquement acceptable.

9. Composé selon la revendication 7 pour l'utilisation dans l'élimination des cellules infectées par le VIH latent.

10. Composé selon la revendication 7 pour l'utilisation dans le traitement d'une infection par le VIH.

11. Composé selon la revendication 1 ou un de ses sels pharmaceutiquement acceptables pour l'utilisation dans la thérapie médicale.

12. Composé selon la revendication 1 ou un de ses sels pharmaceutiquement acceptables et un ou plusieurs agents supplémentaires actifs contre le VIH pour l'utilisation dans l'élimination des cellules infectées par le VIH.
